# EUROPÄISCHE PATENTSCHRIFT

(11) **EP 1 385 943 B1**
(45) Veröffentlichungstag und Bekanntmachung des Hinweises auf die Patenterteilung: **27.06.2007**
(21) Anmeldenummer: 02730196.9
(22) Anmeldetag: 24.04.2002
(51) Int. Cl.: C12N 9/54, C12N 15/31, C12N 5/10, C11D 9/40, C11D 3/386, C12S 9/00, C12S 11/00

(54) **ALKALISCHE PROTEASE-VARIANTEN UND WASCH- UND REINIGUNGSMITTEL ENTHALTEND DIESE ALKALISCHEN PROTEASE-VARIANTEN**
NOVEL ALKALINE PROTEASE VARIANTS AND DETERGENTS AND CLEANING AGENTS CONTAINING SAID NOVEL ALKALINE PROTEASE VARIANTS
NOUVELLES VARIANTES DE PROTEASES ALCALINES ET DETERGENTS CONTENANT CES NOUVELLES VARIANTES

(30) Priorität: 02.05.2001 DE 10121463
(43) Veröffentlichungstag der Anmeldung: 04.02.2004
(73) Patentinhaber: Henkel Kommanditgesellschaft auf Aktien, 40589 Düsseldorf (DE)
(72) Erfinder: KOTTWITZ, Beatrix, 40593 Düsseldorf (DE); MAURER, Karl-Heinz, 40699 Erkrath (DE); BREVES, Roland, 40822 Mettmann (DE)
(86) Internationale Anmeldenummer: PCT/EP2002/004489
(87) Internationale Veröffentlichungsnummer: WO 2002/088340

(56) Entgegenhaltungen:
- EP-A- 0 283 075
- WO-A-89/06279
- WO-A-95/23221

### SEQUENZPROTOKOLL

<110> Henkel Kommanditgesellschaft auf Aktien
<120> Neue Alkalische Protease-Varianten und Wasch- und Reinigungsmittel enthaltend diese neuen Alkalischen Protease-Varianten
<130> H 4727 PCT
<140>
   <141>
<150> DE 10121463.4-41
   <151> 2001-05-02
<160> 4
<170> Patent In Ver. 2.1
<210> 1
   <211> 1773
   <212> DNA
   <213> Bacillus licheniformis ATCC 68614
<220>
   <221> CDS
   <222> (233)..(1375)
<220>
   <221> mat_peptide
   <222> (566)..(1375)
<400> 1
   ggatcctcgg gacctctttc cctgccaggc tgaagcggtc tattcatact ttcgaactga 60
   acatttttct aaaacagtta ttaataacca aaaaatttta aattggtcct ccaaaaaaat 120
   aggcctacca tataattcat tttttttcta taataaatta acagaataat tggaatagat 180
   tatattatcc ttctatttaa attattctga ataaagagga ggagagtgag ta atg atg 238
                                                             Met Met
                                                                 -110
   agg aaa aag agt ttt tgg ctt ggg atg ctg acg gcc ttc atg ctc gtg    286
   Arg Lys Lys Ser Phe Trp Leu Gly Met Leu Thr Ala Phe Met Leu Val
                   -105                -100                -95
   ttc acg atg gca tcg atc gca tcg gct gct gag gaa gca aaa gaa aaa    334
   Phe Thr Met Ala Ser Ile Ala Ser Ala Ala Glu Glu Ala Lys Glu Lys
               -90                 -85                 -80
   tat tta att ggc ttt aat gag cag gaa gct gtc agt gag ttt gta gaa    382
   Tyr Leu Ile Gly Phe Asn Glu Gln Glu Ala Val Ser Glu Phe Val Glu
           -75                 -70                 -65
   caa gta gag gca aat gac gag gtc gcc att ctc tct gag gaa gag gaa    430
   Gln Val Glu Ala Asn Asp Glu Val Ala Ile Leu Ser Glu Glu Glu Glu
       -60                 -55                 -50
   gtc gaa att gaa ctg ctt cat gag ttt gaa acg att cct gtt tta tcc     478
   Val Glu Ile Glu Leu Leu His Glu Phe Glu Thr Ile Pro Val Leu Ser
   -45                 -40                 -35                 -30
   gtt gag tta agc cca gaa gat gtg gac gcg ctt gaa ctt gat cca gcg     526
   Val Glu Leu Ser Pro Glu Asp Val Asp Ala Leu Glu Leu Asp Pro Ala
                   -25                 -20                 -15
   att tct tat att gaa gag gat gca gaa gta acg aca atg gcg caa aca     574
   Ile Ser Tyr Ile Glu Glu Asp Ala Glu Val Thr Thr Met Ala Gln Thr
               -10                  -5                  -1   1
   atc cca tgg gga att agc cgt gtg caa gcc ccg gct gcc cat aac cgt     622
   Ile Pro Trp Gly Ile Ser Arg Val Gln Ala Pro Ala Ala His Asn Arg
         5                  10                  15
   gga ttg aca ggt tct ggt gta aaa gtt gct gtc ctc gat aca ggt att     670
   Gly Leu Thr Gly Ser Gly Val Lys Val Ala Val Leu Asp Thr Gly Ile
    20                  25                  30                  35
   tcc act cat cca gac tta aat att cgt ggt ggc gct agc ttt gta cca     718
   Ser Thr His Pro Asp Leu Asn Ile Arg Gly Gly Ala Ser Phe Val Pro
                    40                  45                  50
   ggg gaa cca tcc act caa gat ggg aat ggg cat ggc acg cat gtg gcc     766
   Gly Glu Pro Ser Thr Gln Asp Gly Asn Gly His Gly Thr His Val Ala
                55                  60                  65
   ggg acg att gct gct tta aac aat tcg att ggc gtt ctt ggc gta gcg     814
   Gly Thr Ile Ala Ala Leu Asn Asn Ser Ile Gly Val Leu Gly Val Ala
            70                  75                  80
   cct agt gcg gaa cta tac gct gtt aaa gtt tta gga gcc gac ggt aga     862
   Pro Ser Ala Glu Leu Tyr Ala Val Lys Val Leu Gly Ala Asp Gly Arg
        85                  90                  95
   ggt gca atc agc tcg att gcc caa ggg ttg gaa tgg gca ggg aac aat     910
   Gly Ala Ile Ser Ser Ile Ala Gln Gly Leu Glu Trp Ala Gly Asn Asn
   100                 105                 110                 115
   ggc atg cac gtt gct aat ttg agt tta gga agc cct tcg cca agt gcc     958
   Gly Met His Val Ala Asn Leu Ser Leu Gly Ser Pro Ser Pro Ser Ala
                   120                 125                 130
   aca ctt gag caa gct gtt aat agc gcg act tct aga ggc gtt ctt gtt    1006
   Thr Leu Glu Gln Ala Val Asn Ser Ala Thr Ser Arg Gly Val Leu Val
               135                 140                 145
   gta gcg gca tct ggg aat tca ggt gca agc tca atc agc tat ccg gcc    1054
   Val Ala Ala Ser Gly Asn Ser Gly Ala Ser Ser Ile Ser Tyr Pro Ala
           150                 155                 160
   cgt tat gcg aac gca atg gca gtc gga gct act gac caa aac aac aac    1102
   Arg Tyr Ala Asn Ala Met Ala Val Gly Ala Thr Asp Gln Asn Asn Asn
       165                 170                 175
   cgc gcc agc ttt tca cag tat ggc cca ggg ctt gac att atg gca cca    1150
   Arg Ala Ser Phe Ser Gln Tyr Gly Pro Gly Leu Asp Ile Met Ala Pro
   180                 185                 190                 195
   ggg gta aac att cag agc aca tac cca ggt tca acg tat gcc agc tta    1198
   Gly Val Asn Ile Gln Ser Thr Tyr Pro Gly Ser Thr Tyr Ala Ser Leu
                   200                 205                 210
   aac ggt aca tcg atg gct act cct cat gtt gca ggt gca gca gcc ctt    1246
   Asn Gly Thr Ser Met Ala Thr Pro His Val Ala Gly Ala Ala Ala Leu
               215                 220                 225
   gtt aaa caa aag aac cca tct tgg tcc aat gta caa atc cgc aac cat    1294
   Val Lys Gln Lys Asn Pro Ser Trp Ser Asn Val Gln Ile Arg Asn His
           230                 235                 240
   cta aag aat acg gca acg agc tta gga agc acg aac ttg tat gga agc    1342
   Leu Lys Asn Thr Ala Thr Ser Leu Gly Ser Thr Asn Leu Tyr Gly Ser
       245                 250                 255
   gga ctt gtc aat gca gaa gcg gca aca cgc taa tcaataaaaa aaccgtgtgc 1395
   Gly Leu Val Asn Ala Glu Ala Ala Thr Arg
   260                 265                 270
   gcttaaaggg cacagctttt tttgtgtatg aatcgaaaaa agagaacaga tcgcaggtct 1455
   caaaaatcga gcgtaaaggg ttgtttaaag ctctttacgc tcgcaggtct tatcgctata 1515
   caatggaaaa ttcacgtctt ttgactttca tggcatattt atttaagtat tcgtttgctt 1575
   tttcgtactc tccgtttttc tggtacctcc ttctactatg ggaaaggtct gatcaatgtc 1635
   gaagctgccg ctcaataaca tattctaaca aatagcatat agaaaaagct agtgttttta 1695
   gcactagctt tttcttcatt ctgatgaagg ttgttcaata ttttgaatcc gttccatgat 1755
   cgtcgggtac cgagctct                                               1773
<210> 2
   <211> 380
   <212> PRT
   <213> Bacillus licheniformis ATCC 68614
<400> 2
   Met Met Arg Lys Lys Ser Phe Trp Leu Gly Met Leu Thr Ala Phe Met
    1               5                  10                  15
   Leu Val Phe Thr Met Ala Ser Ile Ala Ser Ala Ala Glu Glu Ala Lys
               20                  25                  30
   Glu Lys Tyr Leu Ile Gly Phe Asn Glu Gln Glu Ala Val Ser Glu Phe
           35                  40                  45
   Val Glu Gln Val Glu Ala Asn Asp Glu Val Ala Ile Leu Ser Glu Glu
       50                  55                  60
   Glu Glu Val Glu Ile Glu Leu Leu His Glu Phe Glu Thr Ile Pro Val
   65                  70                  75                  80
   Leu Ser Val Glu Leu Ser Pro Glu Asp Val Asp Ala Leu Glu Leu Asp
                   85                  90                  95
   Pro Ala Ile Ser Tyr Ile Glu Glu Asp Ala Glu Val Thr Thr Met Ala
               100                 105                 110
   Gln Thr Ile Pro Trp Gly Ile Ser Arg Val Gln Ala Pro Ala Ala His
           115                 120                 125
   Asn Arg Gly Leu Thr Gly Ser Gly Val Lys Val Ala Val Leu Asp Thr
       130                 135                 140
   Gly Ile Ser Thr His Pro Asp Leu Asn Ile Arg Gly Gly Ala Ser Phe
   145                 150                 155                 160
   Val Pro Gly Glu Pro Ser Thr Gln Asp Gly Asn Gly His Gly Thr His
                   165                 170                 175
   Val Ala Gly Thr Ile Ala Ala Leu Asn Asn Ser Ile Gly Val Leu Gly
               180                 185                 190
   Val Ala Pro Ser Ala Glu Leu Tyr Ala Val Lys Val Leu Gly Ala Asp
           195                 200                 205
   Gly Arg Gly Ala Ile Ser Ser Ile Ala Gln Gly Leu Glu Trp Ala Gly
       210                 215                 220
   Asn Asn Gly Met His Val Ala Asn Leu Ser Leu Gly Ser Pro Ser Pro
   225                 230                 235                 240
   Ser Ala Thr Leu Glu Gln Ala Val Asn Ser Ala Thr Ser Arg Gly Val
                   245                 250                 255
   Leu Val Val Ala Ala Ser Gly Asn Ser Gly Ala Ser Ser Ile Ser Tyr
               260                 265                 270
   Pro Ala Arg Tyr Ala Asn Ala Met Ala Val Gly Ala Thr Asp Gln Asn
           275                 280                 285
   Asn Asn Arg Ala Ser Phe Ser Gln Tyr Gly Pro Gly Leu Asp Ile Met
       290                 295                 300
   Ala Pro Gly Val Asn Ile Gln Ser Thr Tyr Pro Gly Ser Thr Tyr Ala
   305                 310                 315                 320
   Ser Leu Asn Gly Thr Ser Met Ala Thr Pro His Val Ala Gly Ala Ala
                   325                 330                 335
   Ala Leu Val Lys Gln Lys Asn Pro Ser Trp Ser Asn Val Gln Ile Arg
               340                 345                 350
   Asn His Leu Lys Asn Thr Ala Thr Ser Leu Gly Ser Thr Asn Leu Tyr
           355                 360                 365
   Gly Ser Gly Leu Val Asn Ala Glu Ala Ala Thr Arg
       370                 375                 380
<210> 3
   <211> 1143
   <212> DNA
   <213> Künstliche Sequenz
<220>
   <223> Bacillus lentus Alkalische Protease S3T/V4I/V199I/L211G
<220>
   <221> CDS
   <222> (1)..(1143)
<220>
   <221> mat_peptide
   <222> (334)..(1143)
<400> 3
   atg atg agg aaa aag agt ttt tgg ctt ggg atg ctg acg gcc ttc atg    48
   Met Met Arg Lys Lys Ser Phe Trp Leu Gly Met Leu Thr Ala Phe Met
       -110                -105                -100
   ctc gtg ttc acg atg gca tcg atc gca tcg gct gct gag gaa gca aaa   96
   Leu Val Phe Thr Met Ala Ser Ile Ala Ser Ala Ala Glu Glu Ala Lys
   -95                 -90                 -85                 -80
   gaa aaa tat tta att ggc ttt aat gag cag gaa gct gtc agt gag ttt   144
   Glu Lys Tyr Leu Ile Gly Phe Asn Glu Gln Glu Ala Val Ser Glu Phe
                   -75                 -70                 -65
   gta gaa caa gta gag gca aat gac gag gtc gcc att ctc tct gag gaa   192
   Val Glu Gln Val Glu Ala Asn Asp Glu Val Ala Ile Leu Ser Glu Glu
               -60                 -55                 -50
   gag gaa gtc gaa att gaa ctg ctt cat gag ttt gaa acg att cct gtt   240
   Glu Glu Val Glu Ile Glu Leu Leu His Glu Phe Glu Thr Ile Pro Val
           -45                 -40                 -35
   tta tcc gtt gag tta agc cca gaa gat gtg gac gcg ctt gaa ctt gat   288
   Leu Ser Val Glu Leu Ser Pro Glu Asp Val Asp Ala Leu Glu Leu Asp
       -30                 -25                 -20
   cca gcg att tct tat att gaa gag gat gca gaa gta acg aca atg gcg   336
   Pro Ala Ile Ser Tyr Ile Glu Glu Asp Ala Glu Val Thr Thr Met Ala
   -15                 -10                  -5                  -1   1
   caa aca atc cca tgg gga att agc cgt gtg caa gcc ccg gct gcc cat   384
   Gln Thr Ile Pro Trp Gly Ile Ser Arg Val Gln Ala Pro Ala Ala His
                   5                  10                  15
   aac cgt gga ttg aca ggt tct ggt gta aaa gtt gct gtc ctc gat aca   432
   Asn Arg Gly Leu Thr Gly Ser Gly Val Lys Val Ala Val Leu Asp Thr
           20                  25                  30
   ggt att tcc act cat cca gac tta aat att cgt ggt ggc gct agc ttt   480
   Gly Ile Ser Thr His Pro Asp Leu Asn Ile Arg Gly Gly Ala Ser Phe
       35                  40                  45
   gta cca ggg gaa cca tcc act caa gat ggg aat ggg cat ggc acg cat   528
   Val Pro Gly Glu Pro Ser Thr Gln Asp Gly Asn Gly His Gly Thr His
    50                  55                  60                  65
   gtg gcc ggg acg att gct gct tta aac aat tcg att ggc gtt ctt ggc   576
   Val Ala Gly Thr Ile Ala Ala Leu Asn Asn Ser Ile Gly Val Leu Gly
                   70                  75                  80
   gta gcg cct agt gcg gaa cta tac gct gtt aaa gtt tta gga gcc gac   624
   Val Ala Pro Ser Ala Glu Leu Tyr Ala Val Lys Val Leu Gly Ala Asp
               85                  90                  95
   ggt aga ggt gca atc agc tcg att gcc caa ggg ttg gaa tgg gca ggg   672
   Gly Arg Gly Ala Ile Ser Ser Ile Ala Gln Gly Leu Glu Trp Ala Gly
           100                 105                 110
   aac aat ggc atg cac gtt gct aat ttg agt tta gga agc cct tcg cca   720
   Asn Asn Gly Met His Val Ala Asn Leu Ser Leu Gly Ser Pro Ser Pro
       115                 120                 125
   agt gcc aca ctt gag caa gct gtt aat agc gcg act tct aga ggc gtt   768
   Ser Ala Thr Leu Glu Gln Ala Val Asn Ser Ala Thr Ser Arg Gly Val
   130                 135                 140                 145
   ctt gtt gta gcg gca tct ggg aat tca ggt gca agc tca atc agc tat    816
   Leu Val Val Ala Ala Ser Gly Asn Ser Gly Ala Ser Ser Ile Ser Tyr
                   150                 155                 160
   ccg gcc cgt tat gcg aac gca atg gca gtc gga gct act gac caa aac    864
   Pro Ala Arg Tyr Ala Asn Ala Met Ala Val Gly Ala Thr Asp Gln Asn
               165                 170                 175
   aac aac cgc gcc agc ttt tca cag tat ggc gcc ggg ctt gac att gtg    912
   Asn Asn Arg Ala Ser Phe Ser Gln Tyr Gly Ala Gly Leu Asp Ile Val
           180                 185                 190
   gca ccc ggg gta aac att cag agc aca tac cca ggt tca acg tat gct    960
   Ala Pro Gly Val Asn Ile Gln Ser Thr Tyr Pro Gly Ser Thr Tyr Ala
       195                 200                 205
   agc gga aac ggt aca tcg atg gct act cct cat gtt gca ggt gca gca    1008
   Ser Gly Asn Gly Thr Ser Met Ala Thr Pro His Val Ala Gly Ala Ala
   210                 215                 220                 225
   gcc ctt gtt aaa caa aag aac cca tct tgg tcc aat gta caa atc cgc    1056
   Ala Leu Val Lys Gln Lys Asn Pro Ser Trp Ser Asn Val Gln Ile Arg
                   230                 235                 240
   aac cat cta aag aat acg gca acg agc tta gga agc acg aac ttg tat    1104
   Asn His Leu Lys Asn Thr Ala Thr Ser Leu Gly Ser Thr Asn Leu Tyr
               245                 250                 255
   gga agc gga ctt gtc aat gca gaa gcg gca aca cgc taa                1143
   Gly Ser Gly Leu Val Asn Ala Glu Ala Ala Thr Arg
           260                 265                 270
<210> 4
   <211> 380
   <212> PRT
   <213> Künstliche Sequenz
   <223> Bacillus lentus Alkalische Protease S3T/V4I/V199I/L211G
<400> 4
   Met Met Arg Lys Lys Ser Phe Trp Leu Gly Met Leu Thr Ala Phe Met
    1              5                   10                  15
   Leu Val Phe Thr Met Ala Ser Ile Ala Ser Ala Ala Glu Glu Ala Lys
               20                  25                  30
   Glu Lys Tyr Leu Ile Gly Phe Asn Glu Gln Glu Ala Val Ser Glu Phe
           35                  40                  45
   Val Glu Gln Val Glu Ala Asn Asp Glu Val Ala Ile Leu Ser Glu Glu
       50                  55                  60
   Glu Glu Val Glu Ile Glu Leu Leu His Glu Phe Glu Thr Ile Pro Val
   65                  70                  75                  80
   Leu Ser Val Glu Leu Ser Pro Glu Asp Val Asp Ala Leu Glu Leu Asp
                   85                  90                  95
   Pro Ala Ile Ser Tyr Ile Glu Glu Asp Ala Glu Val Thr Thr Met Ala
               100                 105                 110
   Gln Thr Ile Pro Trp Gly Ile Ser Arg Val Gln Ala Pro Ala Ala His
           115                 120                 125
   Asn Arg Gly Leu Thr Gly Ser Gly Val Lys Val Ala Val Leu Asp Thr
       130                 135                 140
   Gly Ile Ser Thr His Pro Asp Leu Asn Ile Arg Gly Gly Ala Ser Phe
   145                 150                 155                 160
   Val Pro Gly Glu Pro Ser Thr Gln Asp Gly Asn Gly His Gly Thr His
                   165                 170                 175
   Val Ala Gly Thr Ile Ala Ala Leu Asn Asn Ser Ile Gly Val Leu Gly
               180                 185                 190
   Val Ala Pro Ser Ala Glu Leu Tyr Ala Val Lys Val Leu Gly Ala Asp
           195                 200                 205
   Gly Arg Gly Ala Ile Ser Ser Ile Ala Gln Gly Leu Glu Trp Ala Gly
       210                 215                 220
   Asn Asn Gly Met His Val Ala Asn Leu Ser Leu Gly Ser Pro Ser Pro
   225                 230                 235                 240
   Ser Ala Thr Leu Glu Gln Ala Val Asn Ser Ala Thr Ser Arg Gly Val
                   245                 250                 255
   Leu Val Val Ala Ala Ser Gly Asn Ser Gly Ala Ser Ser Ile Ser Tyr
               260                 265                 270
   Pro Ala Arg Tyr Ala Asn Ala Met Ala Val Gly Ala Thr Asp Gln Asn
           275                 280                 285
   Asn Asn Arg Ala Ser Phe Ser Gln Tyr Gly Ala Gly Leu Asp Ile Val
       290                 295                 300
   Ala Pro Gly Val Asn Ile Gln Ser Thr Tyr Pro Gly Ser Thr Tyr Ala
   305                 310                 315                 320
   Ser Gly Asn Gly Thr Ser Met Ala Thr Pro His Val Ala Gly Ala Ala
                   325                 330                 335
   Ala Leu Val Lys Gln Lys Asn Pro Ser Trp Ser Asn Val Gln Ile Arg
               340                 345                 350
   Asn His Leu Lys Asn Thr Ala Thr Ser Leu Gly Ser Thr Asn Leu Tyr
           355                 360                 365
   Gly Ser Gly Leu Val Asn Ala Glu Ala Ala Thr Arg
       370                 375                 380

## Beschreibung

Die vorliegende Erfindung betrifft neue Alkalische Protease-Varianten, die sich von natürlichen oder modifizierten Subtilisin-Proteasen ableiten. Sie weisen in der Zahlung nach dem Subtilisin aus *Bacillus lentus* gegenüber den bislang bekannten Subtilisinen die beiden Aminosäuren-Positionen 1991 und 211G auf und mindestens eine zur Stabilisierung des Moleküls beitragende Modifizierung, nämlich nach Punktmutation die Aminosäuren Threonin in Position 3 und/oder Isoleucin in Position 4. Besonders bevorzugt ist die Variante *B*. *lentus*-Alkalische Protease S3T/V4I/V199I/L211G. Diese Varianten zeichnen sich anderen Protease-Varianten gegenüber durch einen verbesserten Beitrag zur Waschleistung in Wasch- und Reinigungsmitteln aus. Deshalb betrifft die vorliegende Erfindung über diese Enzyme hinaus deren Einsatzmöglichkeiten in diversen technischen Prozessen und insbesondere Wasch- und Reinigungsmittel mit diesen neuen Alkalischen Protease-Varianten.

Proteasen vom Subtilisin-Typ (Subtilasen, Subtilopeptidasen, EC 3.4. 21. 62) werden aufgrund der katalytisch wirksamen Aminosäuren den Serin-Proteasen zugerechnet. Sie werden natürlicherweise von Mikroorganismen, insbesondere von *Bacillus*-Spezies gebildet und sekretiert. Sie wirken als unspezifische Endopeptidasen, das heißt sie hydrolysieren beliebige Säureamidbindungen, die im Inneren von Peptiden oder Proteinen liegen. Ih⁻ pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegegeben von R. Bott und C. Betzel, New York, 1998. Subtilisine eignen sich für eine Vielzahl von technischen Verwendungsmöglichkeiten, insbesondere als aktive Inhaltsstoffe von Wasch- oder Reinigungsmitteln.

Neben Enzymen wie beispielsweise Amylasen, Lipasen oder Cellulasen werden Proteasen bereits seit Jahrzehnten als aktive Komponenten in Wasch- und Reinigungsmitteln verwendet. Sie verfügen über die Fähigkeit zum Abbau proteinhaltiger Verunreinigungen auf dem Reinigungsgut, wie beispielsweise Textilien oder Geschirr.

Die Hydrolyseprodukte werden aufgrund ihrer höheren Löslichkeit mit der Waschflotte ausgeschwemmt oder von den übrigen Wasch- oder Reinigungsmittel-Bestandteilen angegriffen, gelöst, emulgiert oder suspendiert. Somit können sich Synergieeffekte zwischen den Enzymen und den übrigen Bestandteilen der betreffenden Wasch- und Reinigungsmittel ergeben.

Subtilisine nehmen aufgrund ihrer günstigen enzymatischen Eigenschaften wie Stabilität oder pH-Optimum unter den Wasch- und Reinigungsmittelproteasen eine herausragende Stellung ein. Im folgenden werden die wichtigsten derzeit in Waschmitteln verwendeten Subtilisin-Proteasen aufgeführt, bei denen es sich zum Teil um natürliche Moleküle, zum Teil um Varianten handelt, die von diesen Wildtyp-Enzymen durch Mutagenese abgeleitet sind.

Subtilisin BPN', welches aus *Bacillus amyloliquefaciens,* beziehungsweise *B. subtilis* stammt, ist aus den Arbeiten von Vasantha et al. (1984) in J. Bacteriol. Volume 159, S. 811-819 und von J. A. Wells et al. (1983) in Nucleic Acids Research, Volume 11, S. 7911-7925 bekannt. Durch Punktmutationen in den Loop-Regionen dieses Enzyms erhaltene Varianten mit verringerter Bindung an das Substrat bei gleichzeitig erhöhter Hydrolyserate werden beispielsweise in den Patentanmeldungen WO 95/07991 und WO 95/30010 vorgestellt. Waschmittel mit derartigen BPN'-Varianten werden beispielsweise in der Patentanmeldung WO 95/29979 offenbart.

Zwei der in der vorliegenden Patentanmeldung betrachteten Aminosäurepositionen, nämlich die Positionen 3 und 4, liegen nicht in Loop-Regionen; die Reste 199 und 211 sind mit den Positionen 205, beziehungsweise 217 von BPN' homolog, welche sich, wie beispielsweise in den genannten Anmeldungen beschrieben ist, im 6. Loop des Moleküls befinden. Dieser ist an der Substratbindung beteiligt. In Position 205 befindet sich bei BPN' natürlicherweise ein Isoleucin (I). Es werden in der Anmeldung WO 95/07991 zahlreiche mögliche Aminosäuren vorgeschlagen, gegen die das natürlicherweise in Position 217 befindliche Tyrosin (Y) ausgetauscht werden kann, jedoch nicht in Verbindung mit stabilisierenden Mutationen des Moleküls; falls eine 217G-Variante beansprucht wird, so nur im Zusammenhang mit anderen, katalytisch kompensierenden Punktmutationen in diesem Substrat-bindenden Loop. Die einzigen tatsächlich offenbarten Varianten (S. 14) mit Austauschen in den Positionen 205 und/oder 217 sind solche, in denen beide Reste gegen raumfüllende, meist auch aliphatische Aminosäuren ausgetauscht worden sind. Ebenso verhält es sich mit der Anmeldung WO 95/29979. Die Subtilisine der Anmeldung WO 95/30010 weisen, wenn sie eine Mutation im 6. Loop besitzen, mindestens noch eine weitere in einem anderen Loop auf. Als 217G-Varianten werden beispielsweise Y217G/S188D oder solche mit noch mehr Austauschen in anderen als dem 6. Loop offenbart, deren homologe Aminosäuren in der erfindungsgemäßen Variante unverändert sind.

Subtilisin BPN' dient insbesondere hinsichtlich der Numerierung der Positionen als Referenzenzym der Subtilisine. So werden beispielsweise auch die auf alle Subtilisine bezogenen Punktmutationen der Anmeldung EP 130756 in der Numerierung von BPN' angegeben. Hierunter ist auch die Position 217, die im erfindungsgemäßen Enzym der Position 211 entspricht. Weitere relevante Positionen werden durch diese Schrift nicht vorbeschrieben.

Die Protease Subtilisin Carlsberg wird in den Publikationen von E. L. Smith et al. von 1968 in J. Biol. Chem., Volume 243, S. 2184-2191 und von Jacobs et al. (1985), Nucl. Acids Res., Band 13, S. 8913-8926 vorgestellt. Sie wird natürlicherweise von *Bacillus licheniformis* gebildet und ist unter dem Handelsnamen Alcalase^{®} von der Firma Novozymes A/S, Bagsværd, Dänemark, erhältlich. Durch Punktmutationen erhältliche Varianten davon mit verringerter Bindung an das Substrat bei gleichzeitig erhöhter Hydrolyserate sind beispielsweise aus der Anmeldung WO 96/28566 bekannt. Hierbei handelt es sich wie bei den oben betrachteten BPN'-Anmeldungen um Varianten, in denen Ein- oder Mehrfachaustausche in den Loop-Regionen des Moleküls vorgenommen worden sind; eine Variante 2041/216G (in Carlsberg-Zählung) ist darin nicht vorbeschrieben.

Die Protease PB92 wird natürlicherweise von dem alkaliphilen Bakterium *Bacillus nov.* spec. 92 produziert und ist unter dem Handelsnamen Maxacal^{®} von der Fa. Gist-Brocades, Delft, Niederlande, erhältlich. In ihrer ursprüglichen Sequenz wird sie in der Patentanmeldung EP 283075 beschrieben. Durch Punktmutation erhaltene Varianten dieses Enzyms, die sich für den Einsatz in Wasch- und Reinigungsmitteln eignen, werden in den Anmeldungen WO 94/02618 und EP 328229 offenbart. Aus der ersten besitzt lediglich die Variante L211G/N212D einen mit der hier beanspruchten Variante identischen Austausch; aus der zweiten geht keine relevante Variante hervor.

Die Subtilisine 147 und 309 werden unter den Handelsnamen Esperase^{®}, beziehungsweise Savinase^{®} von der Fa. Novozymes vertrieben. Sie stammen aus *Bacillus clausii*-Stämmen, die mit der Anmeldung GB 1243784 offenbart werden. Durch Punktmutagenese in Hinblick auf den Einsatz in Wasch- und Reinigungsmitteln weiterentwickelte Varianten dieser Enzyme werden beispielsweise in den Anmeldungen WO 89/06279, WO 95/30011, WO 99/27082 und WO 00/37599 offenbart.

Die Anmeldung WO 89/06279 verfolgte das Ziel, für die Protease höhere Oxidationsstabilität, erhöhte Proteolyserate und verbesserte Waschleistung zu erzielen. Aus ihr (S. 14) geht hervor, daß nur Austausche in bestimmten Positionen die physikalischen oder chemischen Eigenschaften der Moleküle Subtilisin 147 oder 309 ändern sollten; hierunter sind nicht die Positionen 3, 4 oder 211 aufgeführt. In der Anmeldung WO 95/30011 werden Varianten von Subtilisin 309 vorgestellt, die in den Loop-Bereichen des Moleküls Punktmutationen aufweisen und damit verringerte Adsorption an das Substrat bei gleichzeitig erhöhter Hydrolyserate zeigen; hierunter sind keine Mutationen in den Positionen 3 oder 4; die einzige tatsächlich mit der Variante der vorliegenden Anmeldung übereinstimmende Punktmutation ist der Austausch L211G, welcher jedoch in keinem Fall mit einem Austausch V1991 korreliert. In der Anmeldung WO 99/27082 werden am Beispiel von Subtilisin 309 Varianten entwickelt, deren Waschleistung dadurch verbessert wird, daß die aktiven Loops durch Insertion von mehr als einer Aminosäure vergrößert werden. Es handelt sich also nicht wie in der vorliegenden Anmeldung um Substitutionen.

Weitere für den Gebrauch in Wasch- und Reinigungsmitteln etablierte Proteasen sind beispielsweise:
- die aus *Bacillus* spec. 164-A1 erhältliche Protease 164-A1 der Firmen Chemgen Corp., Gaithersburg, MD, USA, und Vista Chemical Company, Austin, TX, USA (WO 93/07276);
- die Alkalische Protease aus *Bacillus* sp. PD138 NCIMB 40338 der Fa. Novozymes (WO 93/18140);
- die aus *Bacillus* sp. ferm. BP-3376 stammende Proteinase K-16 der Fa. Kao Corp., Tokyo, Japan, (US 5344770);
- das von Nedkov et al. 1985 in Biol. Chem Hoppe-Seyler, Band 366, S. 421-430 beschriebene Subtilisin DY, welches mit der Anmeldung WO 96/28557 besonders für den Einsatz in Wasch- und Reinigungsmitteln optimiert worden ist, und zwar wiederum über gezielte Punktmutationen in den aktiven Loops, allerdings ohne daß eine Variante V2041 (entsprechend der Position 199 in dem erfindungsgemäßen Enzym) allein oder in Kombination mit anderen Austauschen darunter ist; und
- die von *Thermoactinomyces vulgaris* (Meloun et al., FEBS Lett. 1983, S. 195-200) gebildete und beispielsweise gemäß der Anmeldung WO 96/28558 optimierte Thermitase.

Unter zahlreichen möglichen Varianten von Thermitase wird in der zuletzt aufgeführten Schrift auch die mit dem Austausch L221G (entsprechend der Position 211 im erfindungsgemäßen Enzym) beschrieben. Da das Enzym natürlicherweise in Position 209 (entsprechend 199 im erfindungsgemäßen Enzym) Isoleucin aufweist, sind hiermit zwei der erfindungswesentlichen Aminosäurereste an den entsprechenden Positionen vorbeschrieben (entsprechend 1991/L211G), jedoch ohne das zusätzliche Merkmal, das Molekül in Gegenwart dieser beiden Aminosäuren in diesen Positionen zusätzlich zu stabilisieren. Insbesondere werden keine Stabilisierungen durch Threonin in Position 3 und/oder Isoleucin in Position 4 (gemäß *B*. *lentus*-Alkalische Protease) vorbeschrieben. In den zu diesen beiden Positionen der Alkalischen Protease aus *B. lentus* homologen Positionen 10 und 11 weist die Thermitase jedoch die Aminosäurereste Serin und Arginin (vergleiche Alignment in WO 91/00345) auf.

Außerdem handelt es sich bei Thermitase um ein Molekül, das insgesamt erhebliche Sequenzabweichungen gegenüber den übrigen Subtilisinen aufweist (Meloun et al., S. 198). So beträgt die Homologie zwischen den maturen Proteinen Thermitase und *B. lentus*-Alkalische Protease 45% Identität (62% ähnliche Aminosäuren). Ähnliches trifft auf die Proteinase K zu (WO 96/28556). Deren Homologie zur *B. lentus*-Alkalischen Protease beträgt auf der Ebene der maturen Proteine nur 33% Identität (46% ähnliche Aminosäuren).

Weitere Proteasen werden beispielsweise in den Anmeldungen EP 199404, EP 251446 , WO 91/06637 und WO 95/10591 beschrieben, die von Procter & Gamble Comp., Cincinnati, OH, USA, als "Protease A", "Protease B", "Protease C", beziehungsweise "Protease D" bezeichnet und in Wasch- und Reinigungsmitteln eingesetzt werden können (vergleiche WO 00/47707, S. 73). Die Proteasen der Anmeldung EP 199404 sind verschiedene Varianten, die auf dem Patent EP 130756 beruhen, jedoch keine Variationen in den für die vorliegenden Anmeldung relevanten Positionen aufweisen (vergleiche EP 199404 A2, Sp. 20). In Beispiel 10 des Patents EP 251446 B1 (S. 49) wird gezeigt, daß Varianten Y217G weniger stabil als das Wildtyp-Enzym sind und dieser Austausch deshalb nicht weiterverfolgt. "Proteasen C" zeichnen sich gemäß der Anmeldung WO 91/06637 durch Punktmutationen in den Positionen 123 und 274 aus. Alle Varianten der "Protease D" tragen gemäß WO 95/10591 Mutationen in Position 76, welche in der vorliegenden Protease unverändert und mit der von BPN' identisch ist; das gleiche trifft auf die Anmeldung, beziehungsweise Patente WO 95/10615, US 6017871 und US 6066611 zu.

Weitere bekannte Proteasen sind die unter den Handelsnamen Durazym^{®}, Relase^{®}, Everlase^{®}, Nafizym, Natalase^{®} und Kannase^{®} von der Firma Novozymes, unter den Handelsnamen, Purafect^{®}, Purafect OxP^{®} und Properase^{®} von der Firma Genencor, unter dem Handelsnamen Protosol^{®} von der Firma Advanced Biochemicals Ltd., Thane, Indien und unter dem Handelsnamen Wuxi^{®} von der Firma Wuxi Snyder Bioproducts Ltd., China, erhältlichen Enzyme.

Um die Waschleistung der Subtilisine zu verbessern, wurde für zahlreiche Anmeldungen die Strategie der Insertion zusätzlicher Aminosäuren in die aktiven Loops verfolgt, so neben den bereits genannten beispielsweise auch für die Anmeldungen, die unter den Nummern WO 00/37599, WO 00/37621 bis WO00/37627 und WO 00/71683 bis WO 00/71691 veröffentlicht worden sind.

Eine andere Strategie besteht darin, die Oberflächenladungen der Moleküle zu verändern. So werden beispielsweise in den Anmeldungen WO 91/00334, WO 91/00335, WO 91/00345, EP 479870, EP 945502 oder EP 563103 zahlreiche Aminosäure-Austausche vorgestellt, mit denen der isoelektrische Punkt der Moleküle erhöht oder verringert werden kann. Die Anmeldung WO 00/24924 leitet daraus ein Verfahren zur Identifizierung entsprechend geeigneter Varianten ab. Das gleiche gilt bezüglich der WO 96/34935, gemäß der nach dem gleichen Prinzip auch die Hydrophobizität der Moleküle variiert werden kann.

Eine weitere Strategie, um die Waschleistung der Subtilisine zu verbessern, besteht darin, statistisch in bekannte Moleküle Punktmutationen einzuführen und die erhaltenen Varianten auf ihre Beiträge zur Waschleistung zu überprüfen. Diese Strategie verfolgt beispielsweise das Patent US 5700676; als einzige die vorliegende Erfindung betreffende Position wird darin ein Austausch in Position 217 (in BPN'-Numerierung), jeweils neben mehreren anderen Austauschen beschrieben. Das gleiche trifft auch auf die Patente US 5310675, US 5801038 US 5955340 und die Anmeldungen WO 99/20723 und WO 99/20727 zu. Auch in dem Patent US 4760025 wird als einzige für die vorliegende Erfindung relevante Mutation eine in Position 217 vorgeschlagen, und zwar deshalb, weil dadurch das aktive Zentrum betroffen ist. Daß die anderen erfindungsgemäßen Austausche ein Rolle bezüglich der Waschleistung spielen könnten, wird durch all diese Schriften nicht nahegelegt.

Eine moderne Richtung der Enzymentwicklung besteht darin, Elemente aus bekannten, miteinander verwandten Proteinen über statistische Verfahren zu neuen Enzymen zu kombinieren, die bislang nicht erreichte Eigenschaften aufweisen. Solche Verfahren werden auch unter dem Oberbegriff Directed Evolution zusamengefaßt. Dazu gehören beispielsweise folgende Verfahren: Die StEP-Methode (Zhao et al. (1998), Nat. Biotechnol., Band 16, S. 258-261), Random priming recombination (Shao et al., (1998), Nucleic Acids Res., Band 26, S. 681-683), DNA-Shuffling (Stemmer, W.P.C. (1994), Nature, Band 370, S. 389-391) oder RACHITT (Coco, W.M. et al. (2001), Nat. Biotechnol., Band 19, S. 354-359).

Eine weitere, insbesondere ergänzende Strategie besteht darin, die Stabilität der betreffenden Proteasen zu erhöhen und damit ihre Wirksamkeit zu erhöhen. Solch eine Stabilisierung ist für Proteasen, die in Kosmetika eingesetzt werden, beispielsweise in US 5230891 beschrieben worden. Für Wasch- und Reinigungsmittel sind dagegen Stabilisierungen durch Punktmutationen geläufiger. So können gemäß US 6087315 und US 6110884 Proteasen dadurch stabilisiert werden, daß man bestimmte Tyrosin-Reste gegen andere austauscht. Andere Möglichkeiten sind beispielsweise:
- Austausch bestimmter Aminosäurereste gegen Prolin gemäß EP 583339;
- Einführung polarerer oder geladener Gruppen auf der Oberfläche des Moleküls gemäß EP 995801;
- Veränderung der Bindung von Metallionen, insbesondere der Calcium-Bindungsstellen, beispielsweise gemäß der Lehre der Anmeldungen WO 88/08028 und WO 88/08033;
Weitere Möglichkeiten, Subtilisine, insbesondere solche, die sich von dem von *Bacillus lentus* ableiten, zu stabilisieren, werden in den Patenten US 5340735, US 5500364, US 5985639 und US 6136553 angegeben.

Bei den Alkalischen Proteasen aus *B.lentus* handelt es sich um hochalkalische Proteasen aus *Bacillus species.* Einer dieser Stämme ist unter der Nummer DSM 5483 hinterlegt worden (WO 91/02792, beziehungsweise EP 493398 und US 5352604). Durch Punktmutation zu erhaltende, in Wasch- und Reinigungsmitteln einsetzbare Varianten dieses Enzyms werden in WO 92/21760, WO 95/23221 und WO 98/30669 offenbart.

Das Wildtypenzym stammt aus einem Produzent, der ursprünglich durch ein Screening nach alkaliphilen Bacillus-Stämmen erhalten worden war, und zeigt selbst eine vergleichsweise hohe Stabilität gegenüber Oxidation und dem Einwirken von Detergenzien. In den Anmeldungen WO 91/02792, beziehungsweise EP 493398 und US 5352604, wird dessen heterologe Expression in dem Wirt *Bacillus licheniformis* ATCC 53926 beschrieben. In den Ansprüchen des genannten US-Patents werden die Positionen 208, 210, 212, 213 und 268 als charakteristisch für die *B*. *lentus*-Alkalische Protease bezeichnet, jedoch keine Variante mit Austauschen in den Positionen 61 und 211.

Auch die Anmeldung WO 92/21760 offenbart das Wildtyp-Enzym *B. lentus*-Alkalische Protease unter SEQ ID NO:52 in seiner Aminosäuresequenz und unter SEQ ID NO:106 in seiner Nukleotidsequenz. Darüber hinaus werden in dieser Anmeldung 51 verschiedene Varianten offenbart, die sich in zahlreichen Positionen vom Wildtyp unterscheiden, darunter sind auch S3T, V41 und V199I.

Auch aus den Anmeldungen WO 95/23221 und WO 98/30669 gehen für den Einsatz in Wasch- und Reinigungsmitteln geeignete *B*. *lentus*-Alkalische Protease-Varianten hervor, die in den drei Positionen S3T, V41 und V1991 mit dem erfindungsgemäßen Enzym übereinstimmen. Darüber hinaus besitzen sie alle zwei oder drei weitere Punktmutationen gegenüber dem Wildtyp-Enzym aus *B. lentus* DSM 5483. Teilweise tragen sie eine zusätzliche Mutation in der Position 211, nämlich 211 D (Varianten F49, F54 und F55); folgerichtig werden mit diesen Anmeldungen die Austausche 211 D und 211 E beansprucht.

Wie all diese über einen langen Zeitraum durchgeführten Arbeiten belegen, besteht ein hoher Bedarf an alternativen Proteasen zum Einsatz in Wasch- und Reinigungsmitteln. Jüngste Publikationen, wie beispielsweise die WO 00/71683 bis WO 00/71691 belegen, daß selbst für die seit langem etablierte Familie der Subtilisin-Proteasen nach wie vor ein Optimierungsbedarf hinsichtlich ihrer Verwendbarkeit in Wasch- und Reinigungsmitteln besteht. Dieser geht mit zahlreichen Arbeiten zur Variation in der Aminosäuresequenz der betreffenden Enzyme einher. Allerdings kann man aus den möglicherweise kalkulierbaren enzymatischen Eigenschaften (vergleiche US 5801039, US 5985639 und US 6136553) nicht ohne weiteres auf das Verhalten dieser Enzyme im Kontext einer Wasch- oder Reinigungsmittelrezeptur schließen. Hier spielen weitere Faktoren wie Stabilität gegenüber oxidierenden Agentien, Denaturierung durch Tenside, Faltungseffekte oder erwünschte Synergien mit anderen Inhaltsstoffen eine Rolle.

Die der vorliegenden Erfindung zugrunde liegende Aufgabe bestand darin, Subtilisine aufzufinden, die in technischen Anwendungen verbesserte Leistungen zeigen. Insbesondere sollten solche aufgefunden werden, die die Wasch-, beziehungsweise Reinigungsleistung von Wasch- und/oder Reinigungsmitteln verbessern.

Eine Teilaufgabe hatte darin bestanden, die Proteasen nicht allein hinsichtlich ihrer Hydrolyseaktivität zu verbessern, sondern auch in entsprechenden Wasch- und Reinigungsmittelrezepturen stabil zu erhalten.

Die vorliegende Patentanmeldung hat hinsichtlich dieses Problems die Strategie verfolgt, das Subtilisin aus *Bacillus lentus* DSM 5483 insbesondere gegenüber den in den Anmeldungen WO 91/02792, WO 92/21760 und WO 95/23221 offenbarten Molekülen für den Einsatz in Wasch- und Reinigungsmitteln weiter zu verbessern.

Überraschenderweise wurde gefunden, daß die Aminosäuren Isoleucin und Glycin in den Positionen 199 und 211 einen erhöhten Beitrag zur Waschleistung ergeben und dieser durch die Aminosäuren Threonin und Isoleucin in den Positionen 3 beziehungsweise 4 vermutlich über einen Stabilitätseffekt verstärkt wird.

Erfindungsgemäß wird diese Aufgabe also durch Alkalische Proteasen vom Subtilisin-Typ gelöst, die dadurch gekennzeichnet sind, daß sie gemäß der Zählung des Subtilisins aus *Bacillus lentus* DSM 5483 in Position 199 Isoleucin und in Position 211 Glycin aufweisen, und zusätzlich eine oder beide der Aminosäuren Threonin in Position 3 und Isoleucin in Position 4.

Sie wird ebenfalls durch Subtilisin-Varianten gelöst, die dadurch gekennzeichnet sind, daß sie gemäß der Zählung des Subtilisins aus *Bacillus lentus* DSM 5483 in Position 3 Threonin, in Position 4 Isoleucin, in Position 199 Isoleucin und in Position 211 Glycin aufweisen.

Insbesondere wird sie durch entsprechende Alkalische Proteasen vom Subtilisin-Typ gelöst, die dadurch gekennzeichnet sind, daß sie von einem Bacillus natürlicherweise gebildet werden oder sich von solch einem Subtilisin ableiten lassen, insbesondere von *Bacillus lentus.*

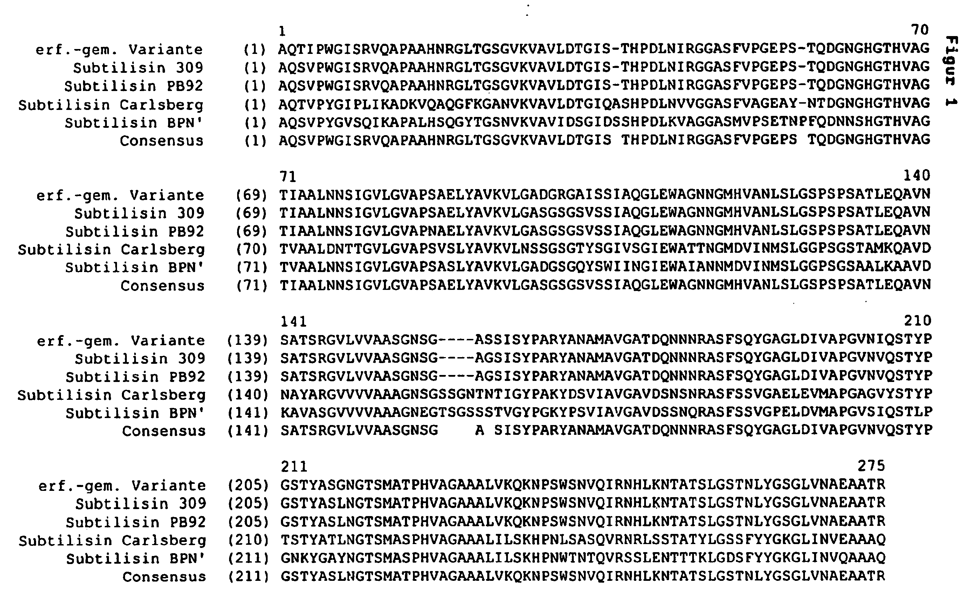

Ganz besonders wird sie von Alkalischen Proteasen gelöst, die von *Bacillus lentus* DSM 5483 natürlicherweise gebildet werden oder sich von solchen Alkalischen Proteasen ableiten lassen, und hierunter insbesondere *B. lentus*-Alkalische Protease S3T/V4I/V199I/L211G gemäß der in Figur 1 (erf. -gem. Variante) angegebenen Aminosäuresequenz.

Als die Variante aus WO 92/21760, die zu der erfindungsgemäßen Variante *B. lentus-*Alkalische Protease S3T/V4I/V199I/L211G das höchste Maß an Übereinstimmung aufweist, muß die *B. lentus*-Alkalische Protease-Variante M131 mit den kennzeichnenden Austauschen S3T/V4I/A188P/V193M/V199I angesehen werden. Sie stimmt in drei Positionen mit denen der erfindungsgemäßen Variante überein. Den Unterschied machen die beiden Austausche A188P und V193M aus, an deren Stelle die erfindungsgemäße Variante mit dem Wildtyp identisch ist. Wie beispielsweise aus der Anmeldung WO 95/30011 zu ersehen ist, liegt die Aminosäure 193 von *B*. *lentus-*Subtilisinen am Anfang des 6. Loops, während die Aminosäure 188 keinem Loop, sondern dem dazwischen liegenden kompakten Bereich des Proteins zuzuordnen ist. Insofern liegen beide Mutationen in strukturell unterschiedlichen Bereichen des Moleküls. Mit der vorliegenden Erfindung wurde überraschenderweise gefunden, daß eine Reversion der beiden Positionen 188 und 193 zu den Aminosäuren des Wildtyps und eine zusätzliche Mutation in der Position 211. also im hinteren Bereich des 6. Loops, ein Enzym ergibt, das den bisher bekannten Enzymen, insbesondere den bislang bekannten Varianten von *B. lentus*-Alkalische Protease in seiner Wasch-, beziehungsweise Reinigungsleistung überlegen ist.

Von den Varianten der Anmeldungen WO 95/23221 und WO 98/30669 unterscheidet sich das erfindungsgemäß besonders bevorzugte Enzym dahingehend, daß mehrere Positionen revertiert, also wieder mit dem Wildtyp identisch sind und sich in Position 211 anstelle des Leucins des Wildtyps oder des Aspartats der genannten Varianten die nicht raumfüllende und ungeladene Aminosäure Glycin befindet.

Aus enzymologischer Sicht ist es überraschend, daß der Effekt der verbesserten Wasch-, beziehungsweise Reinigungsleistung durch einen Austausch in einer Aminosäure erzielt wird, die vermutlich an Substratbindung und/oder Katalyse der Reaktion beteiligt ist; und zwar durch Ersatz der raumfüllenden, hydrophoben Seitenkette des Leucins durch die auf ein Proton reduzierte Seitenkette eines Glycins. Gleichzeitig brauchte dabei die zweite gegenüber dem Wildtyp mutierte Position des 6. Loops, nämlich 199, nicht vom Isoleucin zum Valin des Wildtyps revertiert zu werden. Bei Vergleich mit den Anmeldungen WO 95/23221 oder WO 98/30669 hätte die Punktmutagenese zu einer sauren Gruppe, das heißt L211D oder L211E nähergelegen als die Reversion zur Wildtypsequenz in den anderen mutierten Positionen. Die eingangs zitierten Schriften zur Variation der aktiven Loops der verschiedenen Subtilisine, insbesondere WO 95/30011, hätten bei Variation der Position 211 eine zusätzliche Änderung in einem anderen aktiven Loop nahegelegt, oder innerhalb desselben Loops eine drastischere Änderung, wie beispielsweise V199S/L211D, P204E/L211G oder G196S/L211G, um die Änderung in katalytischer Hinsicht zu kompensieren, keineswegs jedoch das Festhalten an dem Austausch V1991.

Aus der Sicht des anwendungstechnischen Einsatzes, insbesondere in Wasch- und Reinigungsmitteln ist das Überraschende, daß dies zu einer Leistungsverbesserung, insbesondere zu einer Verbesserung des Beitrags derartiger Enzyme zur Wasch- und Reinigungsleistung an verschiedensten Anschmutzungen führt. Der erfolgreiche Einsatz erfindungsgemäßer Subtilisine in entsprechenden Wasch-, beziehungsweise Reinigungsmittelrezepturen (vergleiche Beispiele 2 bis 5) läßt vermuten, daß auch die Stabilität der betreffenden Varianten hoch genug ist, um die Enzyme ausreichend lange aktiv zu halten und somit zur verbesserten Leistung beigetragen hat.

Ein Gegenstand der vorliegenden Erfindung ist eine Alkalische Protease vom Subtilisin-Typ, die dadurch gekennzeichnet ist, daß sie gemäß der Zählung des Subtilisins aus *Bacillus lentus* DSM 5483 in Position 199 Isoleucin und in Position 211 Glycin aufweist und zusätzlich eine der Aminosäuren Threonin in Position 3 oder Isoleucin in Position 4.

Weitere Ausführungsformen dieses Erfindungsgegenstands sind Alkalische Proteasen vom Subtilisin-Typ, die dadurch gekennzeichnet sind, daß sie gemäß der Zählung des Subtilisins aus *Bacillus lentus* DSM 5483 in Position 3 Threonin, in Position 4 Isoleucin, in Position 199 Isoleucin und in Position 211 Glycin aufweisen; daß sie von einem Bacillus, insbesondere von *Bacillus lentus,* natürlicherweise gebildete oder von solch einem Bacillusm abgeleitete Subtilisine sind; daß sie ein von *Bacillus lentus* DSM 5483 natürlicherweise gebildete oder von solch einem abgeleitete Subtilisine sind, insbesondere *B*. *lentus*-Alkalische Protease S3T/V4I/V199I/L211G gemäß der in Figur 1 (erf.-gem Variante) angegebenen Aminosäuresequenz.

Weitere Ausführungsformen dieses Erfindungsgegenstands sind von entsprechenden Alkalischen Proteasen vom Subtilisin-Typ abgeleitete alkalische Proteasen, insbesondere durch Fragmentierung oder Deletionsmutagenese, durch Insertionsmutagenese, durch Substitutionsmutagenese oder durch Fusion mindestens eines Teils mit mindestens einem anderen Protein; solche, die zusätzlich dadurch gekennzeichnet sind, daß sie zusätzlich derivatisiert sind; daß sie eine proteolytische Aktivität, vorzugsweise eine gegenüber dem Ausgangsmolekül, beziehungsweise nicht-derivatisierten Molekül erhöhte proteolytische Aktivität, und ganz besonders eine verbesserte Leistung aufweisen; und/oder daß sie zusätzlich stabilisiert sind.

Einen weiteren Erfindungsgegenstand bilden Nukleinsäuren, die für die im ersten Erfindungsgegenstand bezeichneten alkalischen Proteasen codieren, insbesondere für Subtilisin-Proteasen codierende Nukleinsäuren, deren Nukleotidsequenz mit der in SEQ ID NO. 3 angegebenen Nukleotidsequenz übereinstimmen, besonders in den Bereichen, die für 199 lsoleucin und 211 Glycin und ganz besonders in den Bereichen, die für 3 Threonin, 4 Isoleucin, 199 Isoleucin und 211 Glycin codieren.

Einen weiteren Erfindungsgegenstand bilden Vektoren, die einen oben bezeichneten Nukleinsäurebereich enthalten und insbesondere einen Nukleinsäurebereich enthalten, der für eine der im ersten Erfindungsgegenstand bezeichneten Proteasen oder Derivate codiert. In bevorzugten Ausführungsformen handelt es sich um Klonierungsvektoren, die einen zuvor bezeichneten Nukleinsäurebereich enthalten und insbesondere einen Nukleinsäurebereich enthalten, der für eine der im ersten Erfindungsgegenstand bezeichneten Proteasen oder Derivate codiert; oder um Expressionsvektoren, die einen zuvor bezeichneten Nukleinsäurebereich enthalten und insbesondere einen Nukleinsäurebereich enthalten, der für eine der im ersten Erfindungsgegenstand bezeichneten Proteasen oder Derivate codiert und deren Biosynthese ermöglicht.

Einen weiteren Erfindungsgegenstand bilden Zellen, die einen Vektor nach dem zuvor genannten Erfindungsgegenstand enthalten; die vorzugsweise eine der im ersten Erfindungsgegenstand bezeichneten Proteasen oder Derivate exprimieren oder zu deren Expression angeregt werden können, insbesondere unter Einsatz eines oben bezeichneten Expressionsvektors; die vorzugsweise dadurch gekennzeichnet sind, daß sie Bakterien sind, insbesondere solche, die das gebildete Protein ins umgebende Medium sekretieren; die vorzugsweise dadurch gekennzeichnet ist, daß sie Bakterien der Gattung Bacillus, insbesondere der Species *Bacillus lentus, Bacillus licheniformis, Bacillus amyloliquefaciens, Bacillus subtilis* oder *Bacillus alcalophilus* sind; oder die dadurch gekennzeichnet sind, daß sie eukaryontische Zellen sind, insbesondere solche, die das gebildete Protein posttranslational modifizieren.

Einen weiteren Erfindungsgegenstand bilden Verfahren zur Herstellung eines proteolytischen Enzyms oder Derivats nach dem ersten Erfindungsgegenstand unter Verwendung einer oben bezeichneten Wirtszelle und/oder unter Verwendung eines oben bezeichneten Vektors und/oder unter Verwendung einer oben bezeichneten Nukleinsäure.

Einen weiteren Erfindungsgegenstand bilden Mittel, die dadurch gekennzeichnet sind, daß sie proteolytische Enzyme nach dem ersten Erfindungsgegenstand enthalten, Insbesondere Wasch- oder Reinigungsmittel, ganz besonders in einer Menge von 2 µg bis 20 mg pro g des Mittels; vorzugsweise solche, die dadurch gekennzeichnet sind, daß sie zusätzlich weitere Enzyme, insbesondere andere Proteasen, Amylasen, Cellulasen, Hemicellulasen und/oder Lipasen enthalten.

Einen weiteren Erfindungsgegenstand bilden Mittel zur Behandlung von Textilrohstoffen oder zur Textilpflege, die dadurch gekennzeichnet sind, daß sie allein oder neben anderen aktiven Inhaltsstoffen ein proteolytisches Enzym nach dem ersten Erfindungsgegenstand enthalten, insbesondere für Fasern oder Textilien mit natürlichen Bestandteilen und ganz besonders für solche mit Wolle oder Seide.

Einen weiteren Erfindungsgegenstand bilden Verfahren zur maschinellen Reinigung von Textilien oder von harten Oberflächen, die dadurch gekennzeichnet sind, daß in wenigstens einem der Verfahrensschritte ein proteolytisches Enzym nach dem ersten Erfindungsgegenstand aktiv wird, vorzugsweise in einer Menge von 40 µg bis 4 g, besonders bevorzugt von 400 µg bis 400 mg pro Anwendung.

Einen weiteren Erfindungsgegenstand bilden Verfahren zur Behandlung von Textilrohstoffen oder zur Textilpflege, die dadurch gekennzeichnet sind, daß in wenigstens einem der Verfahrensschritte ein proteolytisches Enzym nach dem ersten Erfindungsgegenstand aktiv wird, insbesondere für Textilrohstoffe oder Textilien mit natürlichen Bestandteilen, insbesondere für solche mit Wolle oder Seide.

Einen weiteren Erfindungsgegenstand bilden Verwendungen eines proteolytischen Enzyms nach dem ersten Erfindungsgegenstand zur Reinigung von Textilien oder von harten Oberflächen, vorzugsweise in einer Menge von 40 µg bis 4 g, besonders bevorzugt von 400 µg bis 400 mg pro Anwendung.

Einen weiteren Erfindungsgegenstand bilden Verwendungen eines proteolytischen Enzyms nach dem ersten Erfindungsgegenstand zur Aktivierung oder Deaktivierung von Inhaltsstoffen von Wasch- oder Reinigungsmitteln.

Einen weiteren Erfindungsgegenstand bilden Verwendungen eines proteolytischen Enzyms nach dem ersten Erfindungsgegenstand zur biochemischen Analyse oder zur Synthese von niedermolekularen Verbindungen oder von Proteinen.

Einen weiteren Erfindungsgegenstand bilden Verwendungen eines proteolytischen Enzyms nach dem ersten Erfindungsgegenstand zur Präparation, Reinigung oder Synthese von Naturstoffen oder biologischen Wertstoffen.

Einen weiteren Erfindungsgegenstand bilden Verwendungen eines proteolytischen Enzyms nach dem ersten Erfindungsgegenstand zur Behandlung von natürlichen Rohstoffen, insbesondere zur Oberflächenbehandlung, ganz besonders in einem Verfahren zur Behandlung von Leder.

Einen weiteren Erfindungsgegenstand bilden Verwendungen eines proteolytischen Enzyms nach einem dem ersten Erfindungsgegenstand zur Gewinnung oder Behandlung von Rohmaterialien oder Zwischenprodukten in der Textilherstellung, insbesondere zum Entfernen von Schutzschichten auf Geweben.

Einen weiteren Erfindungsgegenstand bilden Verwendungen eines proteolytischen Enzyms nach dem ersten Erfindungsgegenstand zur Behandlung von Textilrohstoffen oder zur Textilpflege, insbesondere zur Behandlung von Wolle oder Seide oder woll- oder seidenhaltigen Mischtextilien.

Einen weiteren Erfindungsgegenstand bilden Verwendungen eines proteolytischen Enzyms nach dem ersten Erfindungsgegenstand zur Behandlung von photographischen Filmen, insbesondere zur Entfernung von gelatinhaltigen oder ähnlichen Schutzschichten.

Einen weiteren Erfindungsgegenstand bilden Verwendungen eines proteolytischen Enzyms nach dem ersten Erfindungsgegenstand zur Herstellung von Lebensmitteln oder von Futtermitteln.

Einen weiteren Erfindungsgegenstand bilden Kosmetika mit einem proteolytischen Enzym nach dem ersten Erfindungsgegenstand oder kosmetische Verfahren unter Einbeziehung eines proteolytischen Enzyms nach dem ersten Erfindungsgegenstand oder die Verwendung eines proteolytischen Enzyms nach dem ersten Erfindungsgegenstand zu kosmetischen Zwecken, insbesondere im Rahmen entsprechender Verfahren oder in entsprechenden Mitteln.

Unter einem Protein ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In Tabelle 1 sind die 19 proteinogenen, natürlich vorkommenden L-Aminosäuren zusammengestellt, zusammen mit den 1- und 3-Buchstaben-Codes, mit denen sie auch in der vorliegenden Anmeldung abgekürzt werden.

**Tabelle 1: Die proteinogenen Aminosäuren**

| **1-Buchstaben-Code** | **3-Buchstaben-Code** | **Vollständiger Name** |
|---|---|---|
| A | Ala | Alanin |
| C | Cys | Cystein |
| D | Asp | Asparaginsäure |
| E | Glu | Glutaminsäure |
| F | Phe | Phenylalanin |
| G | Gly | Glycin |
| H | His | Histidin |
| I | Ile | Isoleucin |
| K | Lys | Lysin |
| L | Leu | Leucin |
| M | Met | Methionin |
| N | Asn | Asparagin |
| P | Pro | Prolin |
| Q | Gln | Glutamin |
| R | Arg | Arginin |
| S | Ser | Serin |
| T | Thr | Threonin |
| V | Val | Valin |
| W | Trp | Tryptophan |

Die Kombination einer dieser Bezeichnungen mit einer Nummer bezeichnet für das jeweilige Protein, welchen Aminosäure-Rest es in der jeweiligen Position trägt. So steht S3, beispielsweise für einen Serin-Rest in der Position 3, beginnend mit der Zählung am N-Terminus des betreffenden Proteins. Eine Punktmutation an dieser Stelle, beispielsweise gegen die Aminosäure Threonin wird gemäß dieser Nomenklatur mit der Bezeichnung S3T abgekürzt. Zur Bezeichnung von Varianten mit mehreren Punktmutationen werden diese Austausche mit Schrägstrichen voneinander abgesetzt.

Die Variante S3TN41 ist demnach dadurch gekennzeichnet, daß bei ihr das zuvor in Position 3 vorhandene Serin gegen ein Threonin und das Valin in Position 4 gegen ein Isoleucin ausgetauscht worden ist.

Die Positionsangaben der vorliegenden Erfindnung beziehen sich, soweit nicht anders angegeben, auf die jeweils maturen Formen der betreffenden Proteine, also ohne die Signalpeptide (siehe unten).

Unter einem Enzym ist im Sinne der vorliegenden Anmeldung ein Protein zu verstehen, das eine bestimmte biochemische Funktion ausübt. Beispielsweise unter proteolytischen Enzymen oder Enzymen mit proteolytischer Funktion sind im allgemeinen solche zu verstehen, die die Säureamidbindungen von Proteinen hydrolysieren, insbesondere solche, die im Inneren der Proteine liegen, und deshalb auch als endo-Peptidasen bezeichnet werden können. Subtilisin-Proteasen sind solche endo-Peptidasen, die natürlicherweise von gram-positiven Bakterien gebildet und zumeist sekretiert werden, oder von diesen, beispielsweise über molekularbiologische Methoden abgeleitet sind und sich über Teilbereiche, wie strukturbildende oder funktionstragende Regionen mit den natürlichen Subtilisin-Proteasen homologisieren lassen. Sie werden beispielsweise in dem Artikel "Subtilases: Subtilisin-like Proteases" von R. Siezen, Seite 75-95 in "Subtilisin enzymes", herausgegegeben von R. Bott und C. Betzel, New York, 1996, dargestellt.

Zahlreiche Proteine werden als sogenannte Präproteine, also zusammen mit einem Signalpeptid gebildet. Darunter ist dann der N-terminale Teil des Proteins zu verstehen, dessen Funktion zumeist darin besteht, die Ausschleusung des gebildeten Proteins aus der produzierenden Zelle in das Periplasma oder das umgebende Medium und/oder dessen korrekte Faltung zu gewährleisten. Anschließend wird das Signalpeptid unter natürlichen Bedigungen durch eine Signalpeptidase vom übrigen Protein abgespalten, so daß dieses seine eigentliche katalytische Aktivität ohne die zunächst vorhandenen N-terminalen Aminosäuren ausübt. Gemäß Figur 1 in WO 91/02792 enthält das Präprotein des Subtilisins aus *Bacillus lentus* DSM 5483 380 Aminosäuren. Das mature Protein dagegen nur 269; die Zählung beginnt mit der ersten Aminosäure des maturen Proteins, in diesem Fall also mit dem Alanin, dem nach der Sequenz des Präproteins die Nummer 112 zukommen würde. Gemäß SEQ ID NO. 1 und 2 ist das Signalpeptid des Subtilisins aus *B*. *licheniformis* ATCC 68614 111 Aminosäuren lang und das mature Peptid 269 Aminosäuren. Ohne diese Unterteilung besitzt das vollständige Protein eine Länge von 380 Aminosäuren, wie aus SEQ ID NO. 2 hervorgeht. Das gleiche trifft gemäß SEQ ID NO. 3 und 4 auf die besonders bevorzugte Ausführungsform zu.

Für technische Anwendungen sind aufgrund ihrer enzymatischen Aktivität die maturen Peptide, das heißt die nach ihrer Herstellung prozessierten Enzyme gegenüber den Präproteinen bevorzugt.

Pro-Proteine sind inaktive Vorstufen von Proteinen. Deren Vorläufer mit Signalsequenz werden als Prä-Pro-Proteine bezeichnet.

Unter Nukleinsäuren sind im Sinne der vorliegenden Anmeldung die natürlicherweise aus Nukleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nukleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen.

Die einem Protein entsprechende Informationseinheit einer Nukleinsäure wird auch im Sinne der vorliegenden Anmeldung als Gen bezeichnet. Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen vollständige Gene herzustellen. Einen idealen Ausgangspunkt hierfür stellen DNA-Präparationen aus hinterlegten und/oder kommerziell erhältlichen Mikroorganismen dar. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions- Substitutionsmutationen oder Fusionen zu deletions-, insertions- substitutionsmutierten oder Fusionsgenen und auf Proteinebene zu entsprechenden Deletions-, Insertions- oder Substitutionsvarianten, beziehungsweise Fusionsproteinen.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zelllinie über mehrere Generationen oder Zellteilungen hinweg als vom übrigen Genom unabhängig replizierendes, stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfülllen, das interessierende Gen in der Wirtszelle zu realisieren, das heißt, die Expression des betreffenden Proteins zu ermöglichen. Diese Vektoren werden als Expressionsvektoren bezeichnet.

Durch Homologisierung, das heißt Vergleich mit bekannten Enzymen, wie er beispielsweise über ein Alignment vorgenommen wird, läßt sich aus der Aminosäure- oder Nukleotid-Sequenz die enzymatische Aktivität eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.

Unter dem Begriff eines proteolytischen Enzyms oder dem einer Protease ist deshalb über die Funktionen der wenigen Aminosäurereste des katalytisch aktiven Zentrums hinaus alle Funktionen zu verstehen, wie sie sich durch das Einwirken des gesamten übrigen Proteins oder eines Teils oder mehrerer Teile des übrigen Proteins auf die eigentlich katalytisch aktiven Bereiche ergeben. Auch allein solche modifizierenden Funktionen oder Teilaktivitäten werden, sofern sie eine Proteolyse-Reaktion unterstützen, im Sinne der Erfindung als proteolytische Aktivität angesehen. Zu solchen Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt. Die zweite Voraussetzung dafür, daß es sich um ein erfindungsgemäßes Protein handelt, ist allerdings, daß sich durch das chemische Verhalten der eigentlich aktiven Reste allein oder zusätzlich durch das Einwirken der modifizierenden Teile eine Hydrolyse von Peptid-Bindungen ergibt. Es ist darüberhinaus möglich, daß auch die Aktivitäten anderer Proteasen durch einen oder mehrere Teile, beispielsweise des erfindungsgemäßen Proteins qualitativ oder quantitativ modifiziert werden. Diese Beeinflussung anderer Faktoren wird als proteolytische Aktivität angesehen. Proteolytisch aktive Enzyme sind auch solche, deren Aktivität zu einem gegebenen Zeitpunkt, etwa duch einen Inhibitor blockiert ist. Entscheidend ist ihre prinzipielle Eignung zur entsprechenden Proteolyse-Reaktion.

Unter Fragmenten werden alle Proteine oder Peptide verstanden, die kleiner sind als natürliche Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Unter chimären oder hybriden Proteinen sind im Sinne der vorliegenden Anmeldung solche Proteine zu verstehen, die aus Elementen zusammengesetzt sind, die natürlicherweise von verschiedenen Polypeptidketten aus demselben Organismus oder aus verschiedenen Organismen stammen. Dieses Vorgehen wird auch Shuffling oder Fusionsmutagenese genannt. Der Sinn einer solchen Fusion kann beispielsweise darin bestehen, mithilfe des heranfusionierten erfindungsgemäßen Proteinteils eine enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können. Zur Realisierung der letztgenannten Alternative ist es beispielsweise möglich, posttranslational oder erst nach einem Aufreinigungsschritt durch eine gezielte proteolytische Spaltung eine einzelne chimäre Polypeptidkette in mehrere zu zerlegen.

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können technisch molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifikationen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Ebenso ist unter Derivatisierung die kovalente Bindung an einen makromolekularen Träger zu verstehen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Unter der Leistung eines Enzyms wird dessen Wirksamkeit im jeweils betrachteten technischen Bereich verstanden. Diese basiert auf der eigentlichen enzymatischen Aktivität, hängt darüberhinaus aber von weiteren, für den jeweiligen Prozeß relevanten Fatoren ab. Dazu gehören beispielsweise Stabilität, Substratbindung, Wechselwirkung mit dem das Substrat tragende Material oder Wechselwirkungen mit anderen Inhaltsstoffen, insbesondere Synergien.

Unter der Waschleistung oder der Reinigungsleistung eines Mittels ist im Sinne der vorliegenden Anmeldung der Effekt zu verstehen, den das betrachtete Mittel auf die verschmutzen Artikel, beispielsweise Textilien oder Gegenstände mit harten Oberflächen ausübt. Einzelne Komponenten solcher Mittel, beispielsweise einzelne Enzyme, werden hinsichtlich ihres Beitrags zur Wasch- oder Reinigungsleistung des gesamten Mittels beurteilt. Denn aus den enzymatischen Eigenschaften eines Enzyms kann nicht ohne weiteres auf seinen Beitrag zur Waschleistung eines Mittels geschlossen werden. Hier spielen als weitere Faktoren beispielsweise Stabilität, Substratbindung, Bindung an das Reinigungsgut oder Wechselwirkungen mit anderen Inhaltsstoffen der Mittel, insbesondere Synergien bei der Entfernung der Verschmutzungen eine Rolle.

Gemäß dem Budapester Vertrag über die internationale Anerkennung der Hinterlegung von Mikroorganismen vom 28. April 1977 ist am 10.8.1989 im Zusammenhang mit der Anmeldung WO 91/02792 folgender Mikroorganismus bei der Deutschen Sammlung von Mikroorganismen und Zellkulturen GmbH in Braunschweig (DSMZ) hinterlegt worden: *Bacillus lentus* DSM 5483. Er trägt dort die Registrierungsnummer DSM 5483 (PA5-A0155, Strain 2-1). Die maßgeblichen Angaben über die Merkmale dieses biologischen Materials, werden in WO 91/02792, Tabelle 1 (Seiten 5 bis 7) zusammengestellt. Die DNA- und Aminosäuresequenz der für die vorliegende Anmeldung besonders relevanten Alkalischen Protease aus diesem Organismus gehen aus SEQ ID NO:106, beziehungsweise SEQ ID NO:52 hervor.

Besonders erfindungswesentlich sind die Positionen 3, 4, 199 und 211 der maturen Proteine gemäß der Zählung des Subtilisins aus *Bacillus lentus* DSM 5483 (WO 92/21760). Diese lassen sich gemäß Tabelle 2 mit denen aus den wichtigsten Subtilisinen homologisieren; diese Homologisierung läßt sich auf alle anderen Subtilisine übertragen. So ist beispielsweise in dem Artikel "Subtilases: Subtilisin-like Proteases" von R. Siezen, Seite 75-95 in "Subtilisin enzymes", herausgegegeben von R. Bott und C. Betzel, New York, 1996, ein Alignment von über 20 Subtilisinen relativ zur der bekannten Sequenz von Subtilisin BPN' dargestellt.

**Tabelle 2: Homologisierung der vier besonders erfindungswesentlichen Positionen.**

| **Referenzenzyme** | **Numerierung gemäß der Sequenzen in** | **Pos. 3** | **Pos. 4** | **Pos. 199** | **Pos. 211** |
|---|---|---|---|---|---|
| ***B. lentus-*Alkalische Protease** | WO 92/21760 | S 3 | V 4 | V 199 | L 211 |
| **BPN'** | Wells et al. (siehe oben) | S 3 | V 4 | I 205 | Y 217 |
| **Subtilisin Carlsberg** | Smith et al. (siehe oben) | T 3 | V 4 | V 204 | L 216 |
| **PB92** | EP 283075 | S 3 | V 4 | V 199 | L 211 |
| **Subtilisin 309** | WO 89/06279 | S 3 | V 4 | V 199 | L 211 |
| **Thermitase** | WO 91/00345 | S 10 | R 11 | I 209 | L 221 |
| **Proteinase K** | WO 91/00345 | T 4 | A 6 | I 208 | I 220 |

Ein Alignment der Aminosäuresequenzen einer erfindungsgemäßen *B.lentus-*Alkalischen Protease-Variante mit diesen wichtigsten, einleitend beschriebenen Subtilisinen, nämlich Subtilisin 309 (Savinase^{®}), Subtilisin PB92, Subtilisin Carlsberg und Subtilisin BPN' wird auch in Figur 1 der vorliegenden Patentanmeldung gezeigt.

Aufgrund der hohen strukturellen Übereinstimmungen zwischen den verschiedenen bekannten Subtilisinen und des gleichen von ihnen ausgeübten Reaktionsmechnismus zur Hydrolyse endogener Säureamidbindungen ist zu erwarten, daß die genannten Punktmutationen jeweils im Kontext des betreffenden Moleküls vergleichbare Wirkungen entfalten. Insbesondere ist aufgrund der Lehre der vorliegenden Patentanmeldung zu erwarten, daß solche Subtilisine, die in Stand der Technik bereits in Hinblick auf ihren Einsatz in Wasch- und Reinigungsmitteln entwickelt worden sind durch Übernahme dieser Punktmutationen bezüglich ihrer Beiträge zu den Wasch-, beziehungsweise Reinigungsleistungen weiter verbessert werden.

Besonders die Übernahme der Aminosäuren Isoleucin 199 und Glycin 211 in den homologen Positionen sollte bei aus dem Stand der Technik bekannten Enzymen zu einer Verbesserung ihres Beitrags zur Wasch-, beziehungsweise Reinigungsleistung in entsprechenden Mitteln beitragen. Aufgrund der Erfahrungen an der Alkalischen Protease aus *B. lentus* kommt diesen Austauschen jedoch mindestens eine zusätzliche Stabilisierung der betreffenden Moleküle zugute.

Die Stabilität erfindungsgemäßer Proteasen mit den Aminosäure-Positionen 1991 und 211G kann beispielsweise durch Kopplung an Polymere erhöht werden. Solch eine Methode wird beispielsweise in US 5230891 beschrieben. Sie erfordert, daß die Proteine vor ihrem Einsatz in entsprechenden Mitteln über einen chemischen Kopplungsschritt mit derartigen Polymeren verbunden werden.

Bevorzugt sind Stabilisierungen, die über Punktmutagenese des Moleküls selbst möglich sind. Denn diese erfordern im Anschluß an die Proteingewinnung keine weiteren Arbeitsschritte. Einige hierfür geeigneten Punktmutationen sind an sich aus dem Stand der Technik bekannt. So können gemäß US 6087315 und US 6110884 Proteasen dadurch stabilisiert werden, daß man bestimmte Tyrosin-Reste gegen andere austauscht. Übertragen auf erfindungsgemäße, von *Bacillus lentus* abgeleitete Proteine würde dies Austausche der Tyrosin-Reste in den Positionen 89, 161, 165, 208 und 257 gemäß der SEQ ID NO. 2 bedeuten; die beiden anderen dort angegebenen Positionen werden in *B. lentus*-Alkalische Protease ohnehin schon von Tyrosin eingenommen.

Andere Möglichkeiten sind beispielsweise:
- Der Austausch bestimmter Aminosäurereste gegen Prolin gemäß EP 583339; das würde für Enzyme, die sich von *B. lentus* ableiten, die Austausche S55P, A96P, A166P, A188P und/oder S253P bedeuten);
- die Einführung polarerer oder geladener Gruppen auf der Oberfläche des Moleküls gemäß EP 995801;
- Veränderung der Bindung von Metallionen, insbesondere der Calcium-Bindungsstellen, beispielsweise gemäß der Lehre der Anmeldungen WO 88/08028 und WO 88/08033.
   Gemäß der ersten dieser Schriften müßten eine oder mehrere der an der Calcium-Bindung beteiligten Aminosäure-Reste gegen negativ geladene Aminosäuren ausgetauscht werden. Gemäß der Lehre der zweiten Schrift müßten gleichzeitig in mindestens einer der Folgen der beiden Reste Arginin/Glycin Punktmutationen eingeführt werden; dies betrifft beispielsweise in Subtilisinen aus *Bacillus lentus* die NG-Folgen in den Positionen 60/61, 115/116 und 212/213.
- Gemäß des Patents US 5453372 können Proteine durch bestimmte Mutationen auf der Oberfläche gegen den Einfluß von denaturierenden Agentien wie Tensiden geschützt werden; den dort angegebenen Positionen entsprechen in *B. lentus*-Alkalische Protease die Positionen 134, 155, 158, 164, 188 und/oder 189.
   Weitere vergleichbare Möglichkeiten werden in den Patenten US 5340735, US 5500364, US 5985639 und US 6136553 angegeben.

Erfindungsgemäß erfolgt die Stabilisierung durch die Aminosäurereste von Threonin in Position 3 und/oder von isoleucin in Position 4 gemäß der Zählung des Subtilisins aus *Bacillus lentus* DSM 5483.

Die in den Beispielen der vorliegenden Erfindung untersuchten Varianten lassen vermuten, daß ihre erhöhte Stabilität der ausschlaggebende Faktor ist, um ihnen im Zusammenspiel mit den Aminosäuren 1991 und 211G eine verbesserte Waschleistung zu verleihen.

Unabhängig von dieser Theorie stellen alle Alkalischen Proteasen vom Subtilisin-Typ, die dadurch gekennzeichnet sind, daß sie gemäß der Zählung des Subtilisins aus *Bacillus lentus* DSM 5483 in Position 199 Isoleucin und in Position 211 Glycin aufweisen, und zusätzlich eine der beiden Aminosäuren Threonin in Position 3 und Isoleucin in Position 4 aufweisen, Lösungen der erfindungsgemäßen Aufgabe dar.

Darüberhinaus bevorzugt sind solche Varianten, die zusätzlich zu 199 Isoleucin und 211 Glycin sowohl in Position 3 Threonin als auch in Position 4 Isoleucin aufweisen.

Bevorzugt sind entsprechende Varianten von solchen Alkalischen Proteasen die von einem Bacillus natürlicherweise gebildet werden oder von solch einem abgeleitet sind. Denn Bacillus-Proteasen weisen von vornherein günstige Eigenschaften für diverse technische Einsatzmöglichkeiten auf. Dazu gehören eine gewisse Stabilität gegenüber Temperatur, oxidierenden oder denaturierenden Agentien. Zudem hat man mit mikrobiellen Proteasen die größten Erfahrungen hinsichtlich ihrer biotechnologischen Produktion, was beispielsweise die Konstruktion günstiger Klonierungsvektoren, die Auswahl von Wirtszellen und Fermentationsbedingungen oder die Abschätzung von Risiken, wie beispielsweise die Allergenizität, angeht.

Ganz besonders die Subtilisine aus *Bacillus lentus,* beziehungsweise die Subtilisine, die sich von dessen natürlicherweise gebildeten Proteasen ableiten, sind im Stand der Technik etabliert, beispielsweise für den Einsatz in Wasch- und Reinigungsmitteln. Hierzu gehören die eingangs erwähnten Proteasen Subtilisin 147, Subtilisin 309 und *B*. *lentus*-Alkalische Protease. Der Erfahrungsschatz, den man für die Herstellung und den Einsatz dieser Proteasen erworben hat, kommt erfindungsgemäßen Weiterentwicklungen dieser Enzyme zugute. Hierzu gehört beispielsweise ihre Kompatibilität mit anderen chemischen Verbindungen, wie beispielsweise die Inhaltsstoffe von Wasch- oder Reinigungsmitteln.

Eine besonders bevorzugte Ausführungsform betrifft die erfindungsgemäßen Proteasen, die sich von denen ableiten lassen, die von *Bacillus lentus* DSM 5483 gebildet werden. Hierunter fallen beispielsweise solche der Varianten, die in den Anmeldungen WO 92/21760 oder WO 95/23221 oder WO 98/30669 beschrieben sind. Weiterentwicklungen dieser Enzyme mit den erfindungsgemäßen Substitutionen in den Positionen 199, 211, 3 und/oder 4 kennzeichnen besonders bevorzugte Ausführungsformen der vorliegenden Erfindung.

Die in der vorliegenden Anmeldung untersuchte *B. lentus*-Alkalische Protease S3T/V4I/V199I/L211G ist eine Weiterentwicklung des Subtilisins aus *B. lentus* DSM 5483, das im Sequenz-Protokoll der Anmeldung WO 92/21760 unter SEQ ID NO:52 in seiner Aminosäuresequenz und unter SEQ ID NO:106 in seiner Nukleotidsequenz offenbart ist.

Für diese neue Variante konnte ein höherer Beitrag zur Wasch-, beziehungsweise Reinigungsleistung entsprechender Mittel festgestellt werden, als für die vergleichbaren und im Stand der Technik für diese Zwecke etablierten Enzyme *B*. *lentus*-Alkalische Protease F49 und Savinase^{®} (Beispiele 2-5). Im Sequenzprotokoll ist die Aminosäuresequenz dieser Variante unter der Nummer SEQ ID NO. 2 angegeben. Das für diese Aminosäure-Abfolge codierende Gen ist im Sequenzprotokoll unter der Nummer SEQ ID NO. 1 angegeben. Aufgrund der Degeneriertheit des genetischen Codes sind darüberhinaus zahlreiche andere Nukleinsäuren denkbar, die ebenfalls für diese Variante codieren und gleichermaßen bevorzugte Alternativen innerhalb dieses Erfindungsgegenstands darstellen.

Ein Bakterien-, insbesondere ein Bacillus-Stamm, der die Variante *B. lentus*-Alkalische Protease S3T/V4I/V199I/L211G mit den im Sequenzprotokoll angegebenen DNA- und Aminosäuresequenzen bildet, kann beispielsweise in Anlehnung an das in Beispiel 1 der vorliegenden Anmeldung angegebene Verfahren hergestellt werden.

Von den bislang genannten Alkalischen Proteasen lassen sich über molekularbiologische Methoden, die im Stand der Technik etabliert sind, weitere Proteine ableiten. Solche Methoden werden beispielsweise auch in dem Lehrbuch Fritsch, Sambrook und Maniatis "Molecular cloning: a laboratory manual", Cold Spring Harbour Laboratory Press, New York, 1989, ausführlich behandelt.

Hierzu gehören beispielsweise Varianten, denen über Substitutionsmutagenese, oder über weitere Punktmutationen zusätzliche Eigenschaften verliehen worden sind, die sie hinsichtlich spezifischer Einsatzmöglichkeiten prädestinieren, beispielsweise durch Änderungen der Oberflächenladungen, wie sie in WO 00/36069 offenbart werden, oder durch Veränderungen der an der Katalyse oder Substratbindungen beteiligten Loops, wie sie beispielsweise in WO 99/27082 offenbart werden. Es können auch größere Teilbereiche der Varianten einer Mutagenese unterworfen werden. So kann es beispielsweise das Ziel einer Fragmentbildung oder Deletionsmutagenese sein, spezifische Teilfunktionen der Protease, auszuwählen oder sie im Gegenteil auszuschließen, beispielsweise die Substratbindung, beziehungsweise die über bestimmte Regionen des Moleküls ausgeübten Interaktionen mit anderen Verbindungen.

Durch Insertion, Substitution oder Fusion können erfindungsgemäße Proteasen mit zusätzlichen Funktionen versehen werden. Hierunter ist beispielsweise die Kopplung an bestimmte Domänen denkbar, etwa die Bindung an Cellulose-Bindungs-Domänen, wie sie beispielsweise in den Publikationen WO 99/57154 bis WO 99/57159 beschrieben ist. Die hierin bezeichneten Aminosäure-Linker können verwirklicht werden, indem ein einheitliches Fusionsprotein aus Protease, Linker-Bereich und Bindungsdomäne gebildet wird. Solch eine Bindungsdomäne könnte auch aus derselben oder einer anderen Protease stammen, etwa um die Bindung des erfindungsgemäßen Proteins an ein Protease-Substrat zu verstärken. Dies erhöht die lokale Protease-Konzentration, was in einzelnen Anwendungen, beispielsweise in der Behandlung von Rohstoffen vorteilhaft sein kann.

Die genannten Proteasen können zusätzlich derivatisiert sein, insbesondere um sie für ihren jeweiligen Anwendungszweck zu optimieren. Hierunter fallen chemische Modifikationen, wie sie beispielsweise in der Anmeldung DE 40 13 142 beschrieben sind. Sie können beispielsweise auch durch Kopplung nieder- oder höher-molekularer chemischer Verbindungen modifiziert werden, wie sie natürlicherweise von verschiedenen Organismen im Zusammenhang mit der Proteinbiosynthese durchgeführt werden, wie beispielsweise die Bindung eines Fettsäurerestes nahe des N-Terminus oder Glykosylierungen bei der Synthese durch eukaryontische Wirtzellen. Ausführungsformen der vorliegenden Erfindung stellen somit proteolytische Enzyme oder Fragmente dar, die zusätzlich derivatisiert sind.

Im Zusammenhang mit dem Einsatz erfindungsgemäßer Proteine in Wasch- oder Reinigungsmitteln ist beispielsweise die Kopplung mit anderen waschaktiven Substanzen oder Enzymen besonders sinnvoll. Vergleichbare Kopplungs-Ansätze werden beispielsweise in den Patentanmeldungen WO 00/18865 und WO 00/57155 für Cellulose-Bindungsdomänen beschrieben. Analog dazu können auch Kopplungen an makromolekulare Verbindungen, wie etwa Polyethylenglykol erfolgen, um das Molekül hinsichtlich weiterer Eigenschaften wie Stabilität oder Hautverträglichkeit zu modifizieren.

Solch eine Modifikation wird beispielsweise in dem Patent US 5230891 beschrieben, um die betreffenden Proteasen für den Einsatz in Kosmetika besser geeignet zu machen.

Unter Derivaten erfindungsgemäßer Proteine können im weitesten Sinne auch Präparationen dieser Enzyme verstanden werden. Je nach Gewinnung, Aufarbeitung oder Präparation eines Proteins kann ein solches mit diversen anderen Stoffen vergesellschaftet sein, beispielsweise aus der Kultur der produzierenden Mikroorganismen. Denn bereits Kulturüberstände von proteaseproduzierenden Mikroorganismen zeigen eine proteolytische Aktivität, was zeigt, daß bereits Rohextrakte entsprechende Verwendung finden können, etwa zum Inaktivieren anderer proteinogener Aktivitäten.

Ein Protein kann auch, beispielsweise zur Erhöhung seiner Lagerstabilität, mit bestimmten anderen Stoffen gezielt versetzt worden sein. Erfindungsgemäß sind deshalb auch alle Präparationen des eigentlichen erfindungsgemäßen Proteins. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine proteolytische Funktion entfaltet. Dies kann beispielsweise vom Faltungszustand des Proteins abhängig sein oder aus der reversiblen Bindung eines oder mehrer Begleitstoffe aus der Präparation an ein erfindungsgemäßes Protein. Insbesondere die gemeinsame Präparation von Proteasen mit Protease-Inhibitoren ist aus dem Stand der Technik bekannt (WO 00/01826). Hierunter fallen auch Fusionsproteine, bei denen die Inhibitoren über Linker, insbesondere Aminosäure-Linker an die jeweiligen Proteasen gebunden sind (WO 00/01831).

Besonders erwünscht sind die genannten Weiterentwicklungen, Derivatisierungen und Präparationen erfindungsgemäßer Proteine, wenn sie weiterhin proteolytische Aktivität ausüben, denn das ist die Voraussetzung für ihre erfindungsgemäßen Einsatzmöglichkeiten. Vorzugsweise verfügen die durch alle Arten der Mutagenese und/oder Derivatisierungen erhaltenen Proteasen gegenüber dem Ausgangsmolekül, beziehungsweise dem nicht-derivatisierten Molekül erhöhte proteolytische Aktivität und ganz besonders eine verbesserte Leistungen hinsichtlich ihres jeweils beabsichtigten technischen Einsatzgebietes. Hierzu gehört insbesondere die Verbesserung ihrer Wasch- und/oder Reinigungsleistung zum Einsatz in Wasch- oder Reinigungsmitteln.

Dies ist beispielsweise durch Kombination der erfindungsgemäßen Punktmutationen mit weiteren Punktmutationen möglich, die sich auf die katalysierte Reaktion beziehen, etwa im aktiven Zentrum. So wäre es beispielsweise in Anwendung der Lehre aus der Anmeldung WO 95/30011 möglich, erfindungsgemäße Proteasen, bei denen es sich um solche handelt, die von *Bacillus lentus*-Subtilisin abgeleitet sind, in den Loop-Regionen zu mutieren oder zusätzliche Aminosäuren einzufügen. Derartige Arbeiten sind in den Anmeldungen beschrieben, die unter den Nummern WO 00/37599, WO 00/37621 bis WO 00/37627 und WO 00/71683 bis WO 00/71691 veröffentlicht worden sind.

Die Deletion eines Bereichs des Enzyms, der mit anderen Wirkstoffen im Reaktionsmedium interagiert und somit, etwa über Faltungseffekte, die Gesamtreaktion beeinträchtigt, könnte solch eine gewünschte Weiterentwicklung darstellen. Analog ist die Fusion mit anderen wirksamen Enzymen, etwa mit anderen Proteasen denkbar, um zu einer erhöhten Hydrolyserate zu gelangen.

Beispielsweise die reversible Blockade einer proteolytischen Aktivität während der Lagerung durch Bindung eines Inhibitors kann die Autoproteolyse unterbinden und somit zu einer hohen Proteolyserate zum Zeitpunkt der Verdünnung in dem Reaktionsmedium bewirken. Beispielsweise die Kopplung an spezielle Bindungsdomänen kann bei dem Reinigungsvorgang die Konzentration der Protease nahe des Substrats relativ gegenüber der in der Flotte erhöhen und somit den Beitrag des Enzyms zur Leistung des Mittels erhöhen.

Aus dem Stand der Technik sind zahlreiche Möglichkeiten bekannt, um die Stabilität von Enzymen zu erhöhen, insbesondere von solchen, die in Wasch- und Reinigungsmitteln eingesetzt werden (siehe oben). Hierunter sind aus Praktikabilitätsgründen solche Methoden besonders erfindungsrelevant, die auf Punktmutagenese beruhen. All die oben bereits ausgeführten Möglichkeiten sind auch in Kombination auf erfindungsgemäße Varianten anwendbar. Denn gemäß WO 89/09819 kann davon ausgegangen werden, daß mehrere stabilisierende Mutationen additiv wirken. So können erfindungsgemäße Varianten, die bereits durch eine der beiden Aminosäuren 3T oder 41, oder durch beide stabilisiert sind, durch Kopplung an ein Polymer zusätzlich stabilisiert werden. Sie können aber auch zusätzlich an anderer Stelle des Moleküls eine stabilisierende Mutation aufweisen, beispielsweise durch Substitution eines oder mehrerer der oben bezeichneten Tyrosin-Reste, Einführung bestimmter Prolin-Reste, Veränderung von Oberflächenladungen oder durch Veränderung der Calcium-Bindungsstellen.

Nukleinsäuren bilden den Ausgangspunkt nahezu aller gängigen molekularbiologischen Untersuchungen und Weiterentwicklungen von Proteinen sowie deren Produktion. Dazu gehören insbesondere die Sequenzierung der Gene und die Ableitung der zugehörigen Aminosäure-Sequenz, jede Art von Mutagenese und die Expression der Proteine. Solche Methoden sind, wie bereits oben erwähnt, beispielsweise in dem Handbuch von Fritsch, Sambrook und Maniatis "Molecular cloning: a laboratory manual", Cold Spring Harbour Laboratory Press, New York, 1989, beschrieben. Den zweiten Erfindungsgegenstand bilden somit Nukleinsäuren, die für die Proteine des ersten Erfindungsgegenstands oder deren Derivate codieren.

Auf der Ebene der DNA können die erfindungswesentlichen Enzyme über alle allgemein unter dem Begriff "Protein Engineering" zusammengefaßten Methoden auf verschiedene Anwendungen hin optimiert werden. Insbesondere können dadurch folgende, sich auf der Proteinebene realisierende Eigenschaften erreicht werden: eine Verbesserung der Oxidationsbeständigkeit des abgeleiteten Proteins, der Stabilität gegenüber denaturierenden Agentien oder Proteasen, gegenüber hohen Temperaturen, sauren oder stark Alkalischen Bedingungen, eine Veränderung der Sensitivität gegenüber Calcium oder anderen Cofaktoren, eine Senkung der Immunogenität oder der allergenen Wirkung.

Zu erfindungsgemäßen mutierten Genen gehören beispielsweise solche, die für einzelne, gezielte Basenaustausche oder randomisierte Punktmutationen, für Deletionen einzelner Basen oder von Teilsequenzen, Fusionen mit anderen Genen oder Genfragmenten oder Inversionen verantwortlich sind. Derartige Mutationen oder Modifikationen können das von den betreffenden Nukleinsäuren abgeleitete Enzym für spezifische Anwendungen prädestinieren. Solch eine Mutagenese kann zielgerichtet oder über zufallsgemäße Methoden, beispielsweise mit einem anschließenden auf die Aktivität gerichteten Erkennungs- und/oder Auswahlverfahren (Screening und Selektion) an den klonierten Genen durchgeführt werden.

Insbesondere bei den Nukleinsäuren, die für Proteinfragmente codieren, sind alle drei Leseraster sowohl in *sense-* als auch in *antisense*-Orientierung zu berücksichtigen. Denn solche Oligonukleotide können über die Polymerase-Kettenreaktion (PCR) als Ausgangspunkte zur Synthese verwandter Nukleinsäuren verwendet werden. Solche Oligonukleotide werden, insbesondere, wenn sie einen der Bereiche überdecken, die den vier Aminosäure-Positionen 3, 4, 199 und/oder 211 entsprechen, ausdrücklich in den Schutzbereich der vorliegenden Erfindung einbezogen. Das gilt auch für solche, die gerade in diesen Positionen variable Sequenzen aufweisen, so daß innerhalb einer Population vieler Primer auch mindestens einer vorliegen kann, der für solch eine Position für eine der SEQ ID NO. 3 entsprechenden Teilsequenz codiert. Das gleiche gilt für *Antisense*-Oligonukleotide, die beispielsweise zur Expressions-Regulation eingesetzt werden können.

Die Weiterentwicklung der erfindungsgemäßen Proteasen kann insbesondere an den in der Publikation "Protein engineering" von P.N.Bryan (2000) in Biochim. Biophys. Acta, Band 1543, S. 203-222, vorgestellten Überlegungen ausgerichtet werden.

Bevorzugt sind für Subtilisin-Proteasen codierende Nukleinsäuren, deren Nukleotidsequenz mit der in SEQ ID NO. 3 angegebenen Nukleotidsequenz übereinstimmt- Dies gilt besonders für die Bereiche, die für 199 Isoleucin und 211 Glycin codieren, und ganz besonders für die, die für 3 Threonin. 4 Isoleucin, 199 Isoleucin und 211 Glycin codieren.

Dies gilt vorzugsweise für die, die sich von einer Sequenz für eine *Bacillus lentus-*Protease ableiten lassen, und besonders wenn sie sich von einer Sequenz für eine *Bacillus lentus DSM* 5483-Protease ableiten lassen. Im ganz besonders bevorzugten Fall codiert die Nukleinsäure für die erfindungsgemäße Variante *B. lentus*-Alkalische Protease S3T/V4I/V199I/L211G und/oder stimmt mit der in SEQ ID NO. 3 angegebenen Nukleotidsequenz überein.

In den Schutzbereich sind beispielsweise auch solche Nukleinsäuren einbezogen, die für proteolytisch aktive Insertions- oder Fusionsmutanten codieren. So können die für diese Aktivität verantwortlichen Bereich beispielsweise mit Cellulose bindenden Domänen fusioniert sein oder n katalytisch nicht aktiven Bereichen Punktmutationen tragen, um die Kopplung des abgeleiteten Proteins an ein Polymer zu ermöglichen oder um dessen Allergenizität zu senken.

Um mit den erfindungsrelevanten Nukleinsäuren umzugehen, werden sie geeigneterweise in Vektoren ligiert. Dazu gehören beispielsweise Vektoren, die sich von bakteriellen Plasmiden, von Viren oder von Bacteriophagen ableiten, oder überwiegend synthetische Vektoren. Sie bilden geeignete Ausgangspunkte für molekularbiologische und biochemische Untersuchungen des betreffenden Gens, dessen Expression oder des zugehörigen Proteins. Einen Erfindungsgegenstand der vorliegenden Erfindung stellen somit Vektoren dar, die die oben bezeichneten Nukleinsäuremoleküle enthalten, insbesondere solche, die für die oben bezeichneten proteolytischen Enzyme codieren.

Bevorzugte Ausführungsform dieses Erfindungsgegenstands sind Klonierungsvektoren. Diese eignen sich neben der Lagerung, der biologischen Amplifikation oder der Selektion des interessierenden Gens für die molekularbiologische Charakterisierung des betreffenden Gens. Gleichzeitig stellen sie transportierbare und lagerfähige Formen der beanspruchten Nukleinsäuren dar und sind auch Ausgangspunkte für molekularbiologische Techniken, die nicht an Zellen gebunden sind, wie beispielsweise die PCR oder *In-vitro-*Mutagenese-Verfahren. Bevorzugt sind solche Klonierungsvektoren, die Nukleinsäurebereiche enthalten, die für die oben bezeichneten proteolytischen Enzyme codieren.

Erfindungsgemäße Expressionsvektoren sind die Basis dafür, die Nukleinsäuren des zweiten Erfindungsgegenstands in biologischen Produktionssystemen zu realisieren und damit die Proteine des ersten Erfindungsgegenstands zu produzieren. Bevorzugte Ausführungsformen dieses Erfindungsgegenstands sind Expressionsvektoren, die sämtliche zur Expression notwendigen genetischen Elemente tragen, beispielsweise den natürlichen, ursprünglich vor diesem Gen lokalisierten Promotor oder einen Promotor aus einem anderen Organismus. Diese Elemente können beispielsweise in Form einer sogenannten Expressionskassette angeordnet sein. Bevorzugt sind solche Expressionsvektoren, die Nukleinsäurebereiche enthalten, die für die oben bezeichneten proteolytischen Enzyme codieren.

Eine weitere Umsetzungsmöglichkeit der vorliegenden Erfindung stellen Zellen dar, die einen Vektor nach dem dritten Erfindungsgegenstand enthalten. Sie bilden somit die mikrobiologische Dimension der vorliegenden Erfindung, indem sie beispielsweise die Amplifikation der entsprechenden Gene, aber auch deren Mutagenese oder Transkription und Translation und letztlich die biotechnologische Produktion ermöglichen.

Eine Ausführungsform dieses Erfindungsgegenstands sind Wirtszellen, die eines der erfindungsgemäßen Proteine exprimieren oder zu deren Expression angeregt werden können. Sie ermöglichen damit deren biotechnologische Produktion. Sie müssen dafür das betreffende Gen, geeigneterweise über einen Vektor, erhalten haben, das heißt transformiert worden sein. Dieser Vektor kann in der Wirtszelle extrachromosomal als eigenes genetisches Element vorliegen oder in ein Chromosom integriert sein. Vorzugsweise handelt es sich dabei um einen der oben bezeichneten Expressionsvektoren.

Als Wirtszellen eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Protein kann auf diese Weise aus einer Vielzahl von Wirtsorganismen gewonnen werden.

Bevorzugte Ausführungsformen stellen solche Wirtszellen dar, die aufgrund entsprechender genetischer Elemente in ihrer Aktivität regulierbar sind, beispielsweise durch kontrollierte Zugabe von chemischen Verbindungen, durch Änderung der Kultivierungsbedingungen oder in Abhängigkeit von der jeweiligen Zelldichte. Diese kontrollierbare Expression ermöglicht eine sehr wirtschaftliche Produktion der interessierenden Proteine.

Eine bevorzugte Ausführungsform stellen bakterielle Wirtszellen dar, insbesondere solche, die das gebildete Protein ins umgebende Medium sekretieren. Denn Bakterien zeichnen sich durch kurze Generationszeiten und geringe Ansprüche an die Kultivierungsbedingungen aus. Dadurch können kostengünstige Verfahren etabliert werden. Bei gram-negativen Bakterien, wie beispielsweise *E. coli,* werden eine Vielzahl von Proteinen in den periplasmatischen Raum sekretiert. Dies kann für spezielle Anwendungen vorteilhaft sein. Gram-positive Bakterien, wie beispielsweise Bacilli, geben demgegenüber sekretierte Proteine sogleich in das die Zellen umgebende Nährmedium ab, aus welchem sich nach einer anderen bevorzugten Ausführungsform die exprimierten erfindungsgemäßen Proteine direkt aufreinigen lassen. In der Anmeldung WO 01/81597 wird sogar ein Verfahren offenbart, nach welchem erreicht wird, daß auch gram-negative Bakterien die exprimierten Proteine ausschleusen.

Eine Ausführungsform der vorliegenden Erfindung nutzt *Bacillus lentus* DSM 5483 selbst, um erfindungsgemäße Proteine (homolog) zu exprimieren. Demgegenüber ist jedoch die heterologe Expression bevorzugt. Zu den für die heterologe Expression bevorzugten Bakterien gehören solche der Gattung Bacillus, insbesondere solche der Species *Bacillus licheniformis, Bacillus amyloliquefaciens, Bacillus subtilis* oder andere Species oder Stämme von *Bacillus alcalophilus.* Denn diese bilden vergleichbare Subtilisine selbst, so daß über dieses Verfahren eine Mischung erfindungsmäßer Proteine mit den endogen von den Wirtsstämmen gebildeten Subtilisinen erhalten werden kann. Solch eine Coexpression von *B*. *lentus*-Alkalische Protease in *Bacillus licheniformis* ATCC 53926 wird beispielsweise in der Anmeldung WO 91/02792 (EP 493398 B1) beschrieben; dort finden sich auch zahlreiche mögliche Expressionsvektoren. Es ist zu erwarten, daß sich die neu gefundenen erfindungsgemäßen Varianten, insbesondere die von *Bacillus lentus* und ganz gesonders *B. lentus*-Alkalische Protease S3TN41N1991/L211 G mithilfe dieser Vektoren und/oder in diesem Wirtssystem herstellen lassen.

Eine weitere bevorzugte Ausführungsform dieses Erfindungsgegenstands stellen eukaryontische Wirtszellen dar, insbesondere solche, die das gebildete Protein posttranslational modifizieren. Beispiele für geeignete Eukaryonten sind Pilze wie Actinomyceten oder Hefen wie *Saccharomyces* oder *Kluyveromyces.* Zu den Modifikationen, die derartige Systeme besonders im Zusammenhang mit der Proteinsynthese durchführen, gehören beispielsweise die Bindung niedermolekularer Verbindungen wie Membrananker oder Oligosaccaride. Derartige Oligosaccharid-Modifikationen können beispielsweise zur Senkung der Allergenizität der hergestellten Proteine wünschenswert sein.

Einen eigenständigen Erfindungsgegenstand stellen Verfahren zur Herstellung eines erfindungsgemäßen proteolytischen Enzyms oder Derivats dar. So können beispielsweise anhand der oben bezeichneten DNA- und Aminosäuresequenzen, wie sie beispielsweise auch aus dem Sequenzprotokoll abgeleitet werden können, entsprechende Oligopeptide und Oligonukleotide bis hin zu den vollständigen Genen und Proteinen nach an sich bekannten molekularbiologischen Methoden synthetisiert werden. Ausgehend von den bekannten Subtilisin-produzierenden Mikroorganismen können auch weitere natürliche Produzenten von Subtilisinen isoliert, deren Subtilisin-Sequenzen ermittelt und entsprechend der hier gemachten Vorgaben weiterentwickelt werden. Solche Bakterienspezies können auch für entsprechende Herstellungsverfahren kultiviert und eingesetzt werden. Analog können nach dem Vorbild der beispielsweise in der Anmeldung WO 91/02792 offenbarten Vektoren neue Expressionsvektoren entwickelt werden. Ausführungsformen der vorliegenden Erfindung können auf der Grundlage der zugehörigen Nukleinsäuresequenzen auch zellfreie Expressionssysteme sein, bei denen die Proteinbiosynthese *in vitro* nachvollzogen wird. Alle bereits oben ausgeführten Elemente können auch zu neuen Verfahren kombiniert werden, um erfindungsgemäße Proteine herzustellen. Es ist dabei für jedes erfindungsgemäße Protein eine Vielzahl von Kombinationsmöglichkeiten an Verfahrensschritten denkbar, so das das optimale Verfahren für jeden konkreten Einzelfall experimentell ermittelt werden sollte.

Einen eigenen Erfindungsgegenstand stellen Mittel dar, die dadurch gekennzeichnet sind, daß sie ein erfindungsgemäßes proteolytisches Enzym enthalten.

Praktisch alle technischen Einsatzmöglichkeiten erfindungsgemäßer Enzyme hängen davon ab, daß das funktionsfähige Enzym in einem entsprechenden Medium eingesetzt wird. So verlangen beispielsweise die mikrobiologischen Venrvendungsmöglichkeiten nach Mitteln in denen, das Enzym, meist in Form hochreiner Präparationen mit den notwendigen Reaktionspartnern oder Cofaktoren zusammengebracht wird. Mittel zur Behandlung von Rohmaterialien oder kosmetische Präparationen sind ebenfalls durch spezifische Rezepturen charakterisiert. All diese Rezepturen sollen erfindungsgemäß als Mittel mit dem erfindungsgemäßen Enzym verstanden werden.

Diesem Erfindungsgegenstand werden als bevorzugte Ausführungsform Wasch- oder Reinigungsmittel zugerechnet. Denn, wie in den Ausführungsbeispielen der vorliegenden Anmeldung gezeigt ist, konnte überraschenderweise festgestellt werden, daß eine Subtilisin-Variante 1991/211G (Numerierung gemäß der *B*. *lentus*-Alkalischen Protease), beziehungsweise eine *Bacillus lentus*-Variante mit den Austauschen S3T/V4I/V199I/L211G, insbesondere die Variante *B. lentus*-Alkalische Protease S3T/V4I/V199I/L211G abgeleitet aus *Bacillus lentus* DSM 5483, an diversen Anschmutzungen eine deutliche Leistungssteigerung erbringt (vergleiche Beispiele 2 und 3). Diese übertrifft das Niveau etablierter Waschmittel-Proteasen bei aktivitätsgleichem Einsatz. Das gleiche gilt für entsprechende maschinelle Geschirrspülmittel (vergleiche Beispiel 4 und 5). Dieser Effekt tritt reproduzierbar sowohl bei unterschiedlichen Temperaturen als auch bei unterschiedlichen Konzentrationen ein.

Zu diesem Erfindungsgegenstand zählen alle denkbaren Reinigungsmittelarten, sowohl Konzentrate, als auch unverdünnt anzuwendende Mittel; zum Einsatz im kommerziellen Maßstab, in der Waschmaschine oder bei der Hand-Wäsche, beziehungsweise - Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Reinigungsmittelart stellt eine Ausführungsform der vorliegenden Erfindung dar, sofern sie um ein erfindungsgemäßes Protein bereichert ist.

Ausführungsformen der vorliegenden Erfindung umfassen alle nach den Stand der Technik etablierten und/oder alle zweckmäßigen Darreichungsformen der erfindungsgemäßen Mittel. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Erfindungsgemäße Mittel enthalten erfindungsgemäße Enzyme in einer Menge von 2 µg bis 20 mg und zunehmend bevorzugt von 5 µg bis 17,5 mg, von 20 µg bis 15 mg, von 50 µg bis 10 mg, von 100 µg bis 7,5 mg, von 200 µg bis 5 mg und von 500 µg bis 1 mg pro Gramm des Mittels. Damit ergeben sich von 40 µg bis 4 g und zunehmend bevorzugt von 50 µg bis 3 g, von 100 µg bis 2 g, von 200 µg bis 1 g und besonders bevorzugt von 400 µg bis 400 mg pro Anwendung.

Die Proteaseaktivität in derartigen Mitteln kann nach der in Tenside, Band 7 (1970), S. 125-132 beschriebenen Methode ermittelt werden. Sie wird dementsprechend in PE (Protease-Einheiten) angegeben. Die Proteaseaktivität der Mittel kann bis zu 1.500.000 Proteaseeinheiten pro Gramm Zubereitung betragen.

Neben einem erfindungswesentlichen Enzym enthält ein erfindungsgemäßes Mittel gegebenenfalls weitere Inhaltsstoffe wie Tenside, z. B. nichtionische, anionische und/oder amphotere Tenside, und/oder Bleichmittel, und/oder Builder, sowie gegebenenfalls weitere übliche Inhaltsstoffe.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden kann, sind die Alkylpolyglycoside (APG). Einsetzbare Alkypolyglycoside genügen der allgemeinen Formel RO(G)_{z}, in der R einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Weitere geeignete Tenside sind Polyhydroxyfettsäureamide der Formel (II), 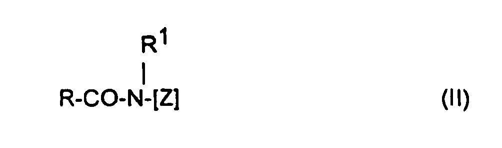 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III),

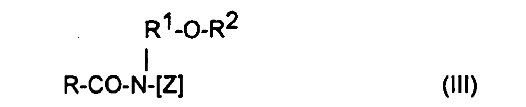

in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierenden Zuckers erhalten, beispielsweise Glucose, Fructose, Maltose, Lactose, Galactose, Mannose oder Xylose. Die N-Alkoxy- oder N-Aryloxy-substituierten Verbindungen können beispielsweise durch Umsetzung mit Fettsäuremethylestern in Gegenwart eines Alkoxids als Katalysator in die gewünschten Polyhydroxyfettsäureamide überführt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, das heißt Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende Alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.-%, üblicherweise von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe oben). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.-% bis 50 Gew.-%, insbesondere von 8 Gew.-% bis 30 Gew.-%, bezogen auf das fertige Mittel, enthalten sein.

Erfindungsgemäße Mittel können Bleichmittel enthalten. Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH₂·H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Mittel an Bleichmittel kann 1 bis 40 Gew.-% und insbesondere 10 bis 20 Gew.-%, betragen, wobei vorteilhafterweise Perboratmonohydrat oder Percarbonat eingesetzt wird.

Um beim Waschen bei Temperaturen von 60 °C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere *n*-Nonanoyl- oder Isononanoyloxybenzolsulfonat (*n*- beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 4443 177 bekannten Kombinationen konventioneller Bleichaktivatoren können eingesetzt werden. Ebenso können Nitrilderivate wie Cyanopyridine, Nitrilquats, zum Beispiel N-Alkylammoniumacetonitrile, und/oder Cyanamidderivate eingesetzt werden. Bevorzugte Bleichaktivatoren sind Natrium-4-(octanoyloxy)-benzolsulfonat, *n*-Nonanoyl- oder Isononanoyloxybenzolsulfonat (*n*- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonüril (MMA). Derartige Bleichaktivatoren können im üblichen Mengenbereich von 0,01 bis 20 Gew.-%, vorzugsweise in Mengen von 0,1 bis 15 Gew.-%, insbesondere 1 Gew.-% bis 10 Gew.-%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren geeignet, wobei solche Verbindungen bevorzugt eingesetzt werden, die in der DE 197 09 284 A1 beschrieben sind. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Erfindungsgemäße Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilicate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 0164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt. Im Handel befinden sich derartige Verbindungen beispielsweise unter der Bezeichnung SKS^{®} (Fa. Clariant). So handelt es sich bei SKS-6^{®} vorwiegend um ein δ-Natriumdisilicat mit der Formel Na₂Si₂O₅·yH₂O, bei SKS-7^{®} vorwiegend um das β-Natriumdisilicat. Durch Reaktion mit Säuren (z.B. Citronensäure oder Kohlensäure) entsteht aus dem δ-Natriumdisilicat Kanemit NaHSi₂O₅·yH₂O, im Handel unter den Bezeichnungen SKS-9^{®} beziehungsweise SKS-10^{®} (Fa. Clariant). Von Vorteil kann es auch sein, chemische Modifikationen dieser Schichtsilicate einzusetzen. So kann beispielsweise die Alkalität der Schichtsilicate geeignet beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂O • y SiO₂ • z P₂O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 196 01 063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, z.B. Citronensäure, beziehungsweise polymeren Polycarboxylaten, z.B. Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein ggf. einsetzbarer, feinkristalliner, synthetischer und gebundenes Wasser enthaltender Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O · Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natrium- beziehungsweise Kaliumtripolyphosphat) in der Wasch- und Reinigungsmittel-Industrie die größte Bedeutung.

Alkalimetallphosphate ist dabei die summarische Bezeichnung für die Alkalimetall-(insbesondere Natrium- und Kalium-) -Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³, Wasserverlust bei 95°), 7 Mol. (Dichte 1,68 gcm⁻³, Schmelzpunkt 48° unter Verlust von 5 H₂O) und 12 Mol. Wasser (Dichte 1,52 gcm⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³ aufweisen. Trinatriumphosphat ist in Wasser unter Alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit Alkalischer Reaktion leicht löslich. Es entsteht z.B. beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°, auch 880° angegeben) und als Decahydrat (Dichte 1,815-1,836 gcm⁻³, Schmelzpunkt 94° unter Wasserverlust). Beide Substanzen sind farblose, in Wasser mit Alkalischer Reaktion lösliche Kristalle. Na₄P₂O₇ entsteht beim Erhitzen von Dinatriumphosphat auf >200° oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natriumbeziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.-%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind erfindungsgemäß genau wie Natriumtripolyphosphat, Kaliumtripolyphosphat oder Mischungen aus diesen beiden einsetzbar; auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, ggf. oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei systemunbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co)polymeren Polycarboxylaten kann von 0,5 bis 20 Gew.-%, insbesondere 1 bis 10 Gew.%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472 042, WO 97/25399, und EP 755 944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolith-, carbonat- und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cobuilder eingesetzt werden.

Buildersubstanzen können in den erfindungsgemäßen Mitteln gegebenenfalls in Mengen bis zu 90 Gew.-% enthalten sein. Sie sind vorzugsweise in Mengen bis zu 75 Gew.-% enthalten. Erfindungsgemäße Waschmittel weisen Buildergehalte von insbesondere 5 Gew.-% bis 50 Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.-% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Lösungsmittel, die in den flüssigen bis gelförmigen Zusammensetzungen von Wasch- und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propyl-ether, Dipropylenglykolmonomethyl-, oder - ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykolt-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.-%, bevorzugt aber unter 15 Gew.-% und insbesondere unterhalb von 10 Gew.-% eingesetzt werden.

Zur Einstellung der Viskosität können der erfindungsgemäßen Zusammensetzung ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickern zählen beispielsweise Polykieselsäuren, Tonmineralien wie Montmorillonite, Zeolithe, Kieselsäuern und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen.

Beispielhaft seien hier Carboxymethylcellulose und andere Celluloseether, Hydroxyethyl- und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.-%, und besonders bevorzugt von 0,1 bis 1,5 Gew.-%, bezogen auf die fertige Zusammensetzung, enthalten sein.

Das erfindungsgemäße Wasch- und Reinigungsmittel kann gegebenenfalls als weitere übliche Inhaltsstoffe Sequestrierungsmittel, Elektrolyte und weitere Hilfsstoffe, wie optische Aufheller, Vergrauungsinhibitoren, Silberkorrosionsinhibitoren, Farbübertragungsinhibitoren, Schauminhibitoren, Abrasivstoffe, Farb- und/oder Duftstoffe, sowie mikrobielle Wirkstoffe und/oder UV-Absorbenzien enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen. Beispiele für derartige Verbindungen sind MnSO₄, V₂O₅, V₂O₄, VO₂, TiOSO₄, K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 16 17 141, beziehungsweise DT 22 00 911). In der deutschen Offenlegungsschrift DT 22 53 063 sind saure Mittel genannt, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyl- oder Ethylgruppenendverschlossene Polyester mit Ethylen- und/oder Propylen-terephthalat- und Polyethylenoxid-terephthalat-Einheiten und Waschmitel, die derartiges Soil-release-Polymer enthalten, bekannt. Das europäische Patent EP 0 241984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272033 sind zumindest anteilig durch C₁₋₄-Alkyl- oder Acylreste endgruppenverschlossene Polyester mit Poly-propylenterephthalat- und Polyoxyethylenterephthalat-Einheiten bekannt. Das europäische Patent EP 0 274 907 beschreibt sulfoethylendgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, ggf. silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, z.B. solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.-%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-ÖI. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Üblicherweise liegt der Gehalt von Wasch- und Reinigungsmitteln an Farbstoffen unter 0,01 Gew.-%, während Duftstoffe bis zu 2 Gew.% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Duftfreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von *K*. *H. Wallhäußer* in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, lsothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, lod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Propanol, 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholinacetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁'-phenyldiguanido-N₅,N₅')-hexan-tetrahydochlorid, 1,6-Di-(N₁,N₁'-phenyl-N₁,N₁-methyldiguanido-N₅,N₅')-hexan-dihydrochlorid, 1,6-Di-(N₁,N₁'-o-chlorophenyldiguanido- N₅,N₅')-hexan-dihydrochlorid, 1,6-Di-(N₁,N₁'-2,6-dichlorophenyldiguanido-N₅,N₅')hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-alpha-methyl-beta.-phenyldiguanido-N₅,N₅')-hexan-dihydrochlorid, 1,6-Di-(N₁,N₁'-p-nitrophenyldiguanido-N₅,N₅')hexan-dihydrochlorid, omega:omega-Di-(N₁,N₁'-phenyldiguanido-N₅,N₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphenyldiguanido- N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅')m-xylene-dihydrochlorid, 1,12-Di-(N₁,N₁'-p-chlorophenyldiguanido-N₅,N₅') dodecan-dihydrochlorid, 1,10-Di-(N₁,N₁'-phenyldiguanido-N₅,N₅')-decan-tetrahydrochlorid, 1,12-Di-(N₁,N₁'-phenyldiguanido- N₅,N₅') dodecantetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyl-trimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormeta-xylol, sowie natürliche antimikrobielle Wirkstoffe pflanzlicher Herkunft (z.B. aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel (R¹)(R²)(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzyl-ammoniumchlorid, CAS No. 8001-54-5), Benzalkon B (*m,p*-Dichlorbenzyl-dimethyl-C12-alkylammoniumchlorid, CAS No. 58390-78-6), Benzoxoniumchlorid (Benzyl-dodecyl-bis-(2-hydroxyethyl)-ammonium-chlorid), Cetrimoniumbromid (N-Hexadecyl-N,N-trimethyl-ammoniumbromid, CAS No. 57-09-0), Benzetoniumchlorid (N,N-Dimethyl-N-[2-[2-[*p-*(1,1,3,3-tetramethylbutyl)-pheno-xy]ethoxy]ethyl]-benzylammoniumchlorid, CAS No. 121-54-0), Dialkyldimethylammonium-chloride wie Di-*n*-decyl-dimethyl-ammoniumchlorid (CAS No. 7173-51-5-5), Didecyldi-methylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethyl-ammoniumchlorid.

Benzalkoniumhalogenide und/oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die antimikrobiellen Wirkstoffe werden in Mengen von 0,0001 Gew.-% bis 1 Gew.-%, bevorzugt von 0,001 Gew.-% bis 0,8 Gew.-%, besonders bevorzugt von 0,005 Gew.-% bis 0,3 Gew.-% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen: 3-Benzylidencampher beziehungsweise 3-Benzylidennorcampher und dessen Derivate, z.B. 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb^{®} HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, das heißt hydrophilisiert oder hydrophobiert vorliegen. Typische Beispiele sind ummantelte Titandioxide, wie z.B. Titandioxid T 805 (Degussa) oder Eusolex^{®} T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet. Weitere geeignete UV-Lichtschutzfilter sind der Übersicht von P. Finkel in SÖFW-Journal 122 (1996), S. 543 zu entnehmen.

Die UV-Absorbenzien werden üblicherweise in Mengen von 0,01 Gew.-% bis 5 Gew.-%, vorzugsweise von 0,03 Gew.-% bis 1 Gew.-%, eingesetzt.

Zu den für Wasch- und Reinigungsmitteln üblichen Inhaltsstoffen werden im allgemeinen auch wasch-, beziehungsweise reinigungsaktive Enzyme gerechnet. Gleichzeitig stellen Wasch- oder Reinigungsmittel, die über ein erfindungsgemäßes Protein hinaus durch zusätzlich weitere Enzyme gekennzeichnet sind, bevorzugte Ausführungsformen der vorliegenden Erfindung dar. Dazu gehören beispielsweise andere Proteasen, aber auch Oxidoreductasen, Cutinasen, Esterasen und/oder Hemicellulasen, und besonders bevorzugt Lipasen, Amylasen, Cellulasen und/oder β-Glucanasen.

Enzyme wie Proteasen, Amylasen, Lipasen oder Cellulasen werden seit Jahrzehnten als aktive Komponenten in Wasch- und Reinigungsmitteln verwendet. Ihr jeweiliger Beitrag zur Wasch-, beziehungsweise Reinigungsleistung des betreffenden Mittels ist im Fall von Protease die Fähigkeit zum Abbau proteinhaltiger Verunreinigungen, im Fall von Amylase der Abbau von stärkehaltigen Verunreinigungen und im Fall von Lipase die fettspaltende Aktivität. Cellulasen werden über ihre schmutzentfernende, das heißt primäre Wasch-, beziehungsweise Reinigungsleistung hinaus, insbesondere wegen ihres Beitrags zur Sekundärwaschleistung eines Waschmittels und wegen ihrer Faserwirkung auf Textilien bevorzugt in Waschmitteln eingesetzt. Die jeweiligen Hydrolyseprodukte werden von den übrigen Wasch- oder Reinigungsmittel-Bestandteilen angegriffen, gelöst, emulgiert oder suspendiert oder aufgrund ihrer höheren Löslichkeit mit der Waschflotte ausgeschwemmt, so daß es zu Synergieeffekten zwischen den Enzymen und den übrigen Bestandteilen kommt.

Einen dem Beitrag zur Sekundärwaschleistung eines Mittels durch Cellulase vergleichbaren Effekt können Proteasen auf natürliche Fasern, insbesondere auf Wolle oder Seide ausüben. Durch ihre Wirkung auf die Oberflächenstruktur solcher Gewebe können sie einen glättenden Einfluß auf das Material ausüben und damit dem Verfilzen entgegenwirken.

Weitere Enzyme erweitern die Reinigungsleistung entsprechender Mittel um ihre jeweils spezifische enzymatische Leistung. Dazu gehören beispielsweise β-Glucanasen (WO 99/06515 und WO 99/06516), Laccasen (WO 00/39306) oder Pektin-lösende Enzyme (WO 00/42145), die insbesondere in Spezialwaschmitteln zum Einsatz kommen.

Zur Verwendung in erfindungsgemäßen Mitteln kommen in erster Linie aus Mikroorganismen, wie Bakterien oder Pilzen, gewonnene Enzyme in Frage. Sie werden in bekannter Weise durch Fermentationsprozesse aus geeigneten Mikroorganismen gewonnen, die zum Beispiel in den deutschen Offenlegungsschriften DE 1940488, und DE 2121397, den US-amerikanischen Patentschriften US 3623957, US 4264738, der europäischen Patentanmeldung EP 006638 sowie der internationalen Patentanmeldung WO 91/02792 beschrieben sind.

Ein erfindungsgemäßes Protein und/oder andere enthaltene Proteine können besonders während der Lagerung durch Stabilisatoren beispielsweise vor Denaturierung, Zerfall oder Inaktivierung, etwa durch physikalische Einflüsse, Oxidation oder proteolytische Spaltung geschützt werden.

Eine Gruppe von Stabilisatoren sind reversible Proteaseinhibitoren, die bei Verdünnung des Mittels in der Waschflotte abdissozieren. Benzamidin-Hydrochlorid und Leupeptin sind für diesen Zweck etabliert. Häufig werden Borax, Borsäuren, Boronsäuren oder deren Salze oder Ester verwendet, darunter vor allem Derivate mit aromatischen Gruppen, etwa gemäß WO 95/12655 ortho-substituierte, gemäß WO 92/19707 metasubstituierte und gemäß US 5 972 873 para-substituierte Phenylboronsäuren, beziehungsweise deren Salze oder Ester. In den Anmeldungen WO 98/13460 und EP 583 534 werden Peptidaldehyde, das heißt Oligopeptide mit reduziertem C-Terminus, und zwar solche aus 2 - 50 Monomeren, zur reversiblen Inhibierung von Wasch- und Reinigungsmittelproteasen offenbart. Zu den peptidischen reversiblen Proteaseinhibitoren gehört u.a. Ovomucoid (WO 93/00418). Beispielsweise mit der Anmeldung WO 00/01826 werden spezifische, reversible Peptid-Inhibitoren für die Protease Subtilisin zur Verwendung in Protease-haltigen Mitteln offenbart, und mit WO 00/01831 entsprechende Fusionsproteine aus Protease und Inhibitor.

Weitere Enzymstabilisatoren sind Aminoalkohole wie Mono-, Di-, Triethanol- und -propanolamin und deren Mischungen, aliphatische Carbonsäuren bis zu C₁₂, wie beispielsweise aus den Anmeldungen EP 0 378 261 und WO 97/05227 bekannt ist, wie Bernsteinsäure, andere Dicarbonsäuren oder Salze der genannten Säuren. Mit der Anmeldung DE 196 50 537 werden für diesen Zweck endgruppenverschlossene Fettsäureamidalkoxylate offenbart. Bestimmte als Builder eingesetzte organische Säuren vermögen, wie in WO 97/18287 offenbart, zusätzlich ein enthaltenes Enzym zu stabilisieren.

Niedere aliphatische Alkohole, v.a. aber Polyole, wie beispielsweise Glycerin, Ethylenglykol, Propylenglykol oder Sorbit sind weitere häufig eingesetzte Enzymstabilisatoren. Ebenso werden Calciumsalze verwendet, wie beispielsweise Calciumacetat oder das in der Patentschrift EP 0 028 865 für diesen Zweck offenbarte Calcium-Formiat, und Magnesiumsalze, etwa gemäß der europäischen Anmeldung EP 0 378 262.

Polyamid-Oligomere (WO 99/43780) oder polymere Verbindungen wie Lignin (WO 97/00932), wasserlösliche Vinyl-Copolymere (EP 828 762) oder, wie in EP 702 712 offenbart, Cellulose-Ether, Acryl-Polymere und/oder Polyamide stabilisieren die Enzym-Präparation u. a. gegenüber physikalischen Einflüssen oder pH-Wert-Schwankungen. Polyamin-N-Oxid-enthaltende Polymere (EP 587 550 und EP 581 751) wirken gleichzeitig als Enzymstabilisatoren und als Farbübertragungsinhibitoren. Andere polymere Stabilisatoren sind die in WO 97/05227 neben anderen Bestandteilen offenbarten linearen C₈-C₁₈ Polyoxyalkylene. Alkylpolyglycoside könnten wie in den Anmeldungen WO 97/43377 und WO 98/45396 die enzymatischen Komponenten des erfindungsgemäßen Mittels stabilisieren und sogar in ihrer Leistung steigern. Vernetzte N-haltige Verbindungen, wie in WO 98/17764 offenbart, erfüllen eine Doppelfunktion als Soil-release-Agentien und als Enzym-Stabilisatoren. Hydrophobes, nichtionisches Polymer wirkt in einer Mischung mit anderen Stabilisatoren gemäß der Anmeldung WO 97/32958 stabilisierend auf eine Cellulase, so daß sich solche oder ähnliche Bestandteile auch für das erfindungswesentliche Enzym eignen könnten.

Reduktionsmittel und Antioxidantien erhöhen, wie u.a. in EP 780 466 offenbart, die Stabilität der Enzyme gegenüber oxidativem Zerfall. Schwefelhaltige Reduktionsmittel sind beispielsweise aus den Patentschriften EP 0 080 748 und EP 0 080 223 bekannt. Andere Beispiele sind Natrium-Sulfit (EP 533 239) und reduzierende Zucker (EP 656 058).

Vielfach werden auch Kombinatonen von Stabilisatoren verwendet, beispielsweise aus Polyolen, Borsäure und/oder Borax in der Anmeldung WO 96/31589, die Kombination von Borsäure oder Borat, reduzierenden Salzen und Bernsteinsäure oder anderen Dicarbonsäuren in der Anmeldung EP 126 505 oder die Kombination von Borsäure oder Borat mit Polyolen oder Polyaminoverbindungen und mit reduzierenden Salzen, wie in der Anmeldung EP 080 223 offenbart. Die Wirkung von Peptid-Aldehyd-Stabilisatoren wird gemäß WO 98/13462 durch die Kombination mit Borsäure und/oder Borsäurederivaten und Polyolen gesteigert und gemäß WO 98/13459 durch die zusätzliche Verwendung von Calcium-lonen noch weiter verstärkt.

Mittel mit stabilisierten Enzymaktivitäten stellen bevorzugte Ausführungsformen der vorliegenden Erfindung dar. Besonders bevorzugt sind solche mit Enzymen, die auf mehrere der dargestellten Weisen stabilisiert sind.

Da erfindungsgemäße Mittel in allen denkbaren Formen angeboten werden können, stellen erfindungsgemäße Enzyme, beziehungsweise Proteine in allen für die Zugabe zu den jeweiligen Mitteln zweckmäßigen Formulierungen jeweilige Ausführungsformen der vorliegenden Erfindung dar. Dazu gehören beispielsweise flüssige Formulierungen, feste Granulate oder Kapseln.

Die verkapselte Form bietet sich an, um die Enzyme oder andere Inhaltsstoffe vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Solche Kapseln werden beispielsweise mit den Patentanmeldungen WO 97/24177 und DE 199 18 267 offenbart. Eine mögliche Verkapselungsmethode besteht darin, daß die Proteine, ausgehend von einer Mischung der Proteinlösung mit einer Lösung oder Suspension von Stärke oder einem Stärkederivat, in dieser Substanz verkapselt werden. Ein solches Verkapselungsverfahren wird mit der deutschen Anmeldung DE 199 56 382 mit dem Titel "Verfahren zur Herstellung von mikroverkapselten Enzymen" beschrieben.

Im Fall fester Mittel können die Proteine beispielsweise in getrockneter, granulierter und/oder verkapselter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Flüssigen, gelförmigen oder pastösen erfindungsgemäßen Mitteln können die Enzyme, und auch das erfindungswesentliche Protein ausgehend von einer nach dem Stand der Technik durchgeführten Proteingewinnung und Präparation in konzentrierter wäßriger oder nichtwäßriger Lösung, Suspension oder Emulsion zugesetzt werden, aber auch in Gelform oder verkapselt oder als getrocknetes Pulver. Derartige erfindungsgemäße Wasch- oder Reinigungsmittel werden in der Regel durch einfaches Mischen der Inhaltsstoffe hergestellt, die in Substanz oder als Lösung in einen automatischen Mischer gegeben werden können.

Neben der primären Waschleistung können die in Waschmitteln enthaltenen Proteasen ferner die Funktion erfüllen, andere enzymatische Bestandteile durch proteolytische Spaltung zu aktivieren oder nach entsprechender Einwirkzeit zu inaktivieren, so wie beispielsweise in den Anmeldungen WO 94/29426 oder EP 747 471 offenbart worden ist. Vergleichbare regulatorische Funktionen sind auch über das erfindungsgemäße Enzym möglich. Eine Ausführungsform der vorliegenden Erfindung sind ferner solche Mittel mit Kapseln aus proteasesensitivem Material, welche beispielsweise von erfindungsgemäßen Proteinen zu einem beabsichtigten Zeitpunkt hydrolysiert werden und ihren Inhalt freisetzen. Ein vergleichbarer Effekt kann auch bei anderen mehrphasigen Mitteln erzielt werden.

Einen eigenen Erfindungsgegenstand stellen Mittel zur Behandlung von Textitrohstoffen oder zur Textilpflege dar, die dadurch gekennzeichnet sind, daß sie allein oder neben anderen Inhaltsstoffen ein erfindungsgemäßes proteolytisches Enzym enthalten. Denn insbesondere natürliche Fasern, wie beispielsweise Wolle oder Seide, zeichnen sich durch eine charakteristische, mikroskopische Oberflächenstruktur aus. Diese kann, wie am Beispiel der Wolle im Artikel von R. Breier in Melliand Textilberichte vom 1.4.2000 (S. 263) ausgeführt worden ist, langfristig zu unerwünschten Effekten, wie etwa Verfilzung führen. Zur Vermeidung solcher Effekte werden die natürlichen Rohstoffe mit erfindungsgemäßen Mitteln behandelt, welche beispielsweise dazu beitragen, die auf Proteinstrukturen beruhende geschuppte Oberflächenstruktur zu glätten und damit einem Verfilzen entgegenwirken. Eine besonders bevorzugte Ausführungsform stellen derartige Mittel für Fasern oder Textilien mit natürlichen Bestandteilen und ganz besonders mit Wolle oder Seide dar.

In einer Ausführungsform der vorliegenden Erfindung ist das Mittel mit einer erfindungsgemäßen Protease so konzipiert, daß es regelmäßig als Pflegemittel verwendet werden kann, beispielsweise indem es dem Waschprozeß zugesetzt, nach dem Waschen angewendet oder unabhängig von dem Waschen appliziert wird. Der gewünschte Effekt besteht darin, eine glatte Oberflächenstruktur des Textils zu erhalten und/oder Schädigungen des Gewebes vorzubeugen und/oder zu verringern.

Einen eigenen Erfindungsgegenstand stellen Verfahren zur maschinellen Reinigung von Textilien oder von harten Oberflächen dar, die dadurch gekennzeichnet sind, daß in wenigstens einem der Verfahrensschritte ein erfindungsgemäßes proteolytisches Enzym aktiv wird.

Verfahren zur maschinellen Reinigung von Textilien zeichnen sich im allgemeinen dadurch aus, daß in mehreren Verfahrensschritten verschiedene reinigungsaktive Substanzen auf das Reinigungsgut aufgebracht und nach der Einwirkzeit abgewaschen werden, oder daß das Reinigungsgut in sonstiger Weise mit einem Reinigungsmittel oder einer Lösung dieses Mittels behandelt wird. Das gleiche gilt für Verfahren zur maschinellen Reinigung von allen anderen Materialien als Textilien, welche unter dem Begriff harte Oberflächen zusammengefaßt werden. Derartige Verfahren können in wenigstens einem der Verfahrensschritte um erfindungsgemäße Proteine bereichert werden, und stellen dann Ausführungsformen der vorliegenden Erfindung dar.

Bevorzugt sind Verfahren, bei denen ein erfindungsgemäßes Enzym in einer Menge von 40 µg bis 4 g und zunehmend bevorzugt von 50 µg bis 3 g, von 100 µg bis 2 g von 200 µg bis 1 g und besonders bevorzugt von 400 µg bis 400 mg pro Anwendung eingesetzt wird.

Da das erfindungsgemäße Enzym natürlicherweise bereits eine proteinauflösende Aktivität besitzt und diese auch in Medien entfaltet, die sonst keine Reinigungskraft besitzen, wie beispielsweise in bloßem Puffer, kann ein einzelner Teilschritt eines solchen Verfahrens zur maschinellen Reinigung von Textilien darin bestehen, daß gewünschtenfalls neben stabilisierenden Verbindungen, Salzen oder Puffersubstanzen als einzige reinigungsaktive Komponente das erfindungsgemäße Enzym aufgebracht wird. Dies stellt eine besonders bevorzugte Ausführungsform der vorliegenden Erfindung dar.

Einen eigenen Erfindungsgegenstand stellen Verfahren zur Behandlung von Textilrohstoffen oder zur Textilpflege dar, die dadurch gekennzeichnet sind, daß in wenigstens einem der Verfahrensschritte ein erfindungsgemäßes proteolytisches Enzym aktiv wird. Es kann sich dabei beispielsweise um Verfahren handeln, in denen Materialien zur Verarbeitung in Textilien vorbereitet werden, etwa zur Antifilzausrüstung, oder beispielsweise um Verfahren, welche die Reinigung getragener Textilien um eine pflegende Komponente bereichern. Wegen der oben beschriebenen Wirkung von Proteasen auf bestimmte Gewebe handelt es sich in bevorzugten Ausführungsformen um Textilrohstoffe oder Textilien mit natürlichen Bestandteilen, insbesondere mit Wolle oder Seide.

Einen eigenen Erfindungsgegenstand stellt die Verwendung eines erfindungsgemäßen proteolytischen Enzyms zur Reinigung von Textilien oder von harten Oberflächen dar. Denn erfindungsgemäße Enzyme können, insbesondere entsprechend den oben beschriebenen Verfahren dazu verwendet werden, um von Textilien oder von harten Oberflächen proteinhaltige Verunreinigungen zu beseitigen. Um bevorzugte Ausführungsformen handelt es sich bei der Verwendung außerhalb eines maschinellen Verfahrens, beispielsweise bei der Handwäsche oder bei der manuellen Entfernung von Flecken von Textilien oder von harten Oberflächen.

Bevorzugt ist die Verwendung eines erfindungsgemäßen Enzyms in einer Menge von 40 µg bis 4 g und zunehmend bevorzugt von 50 µg bis 3 g, von 100 µg bis 2 g von 200 µg bis 1 g und besonders bevorzugt von 400 µg bis 400 mg pro Anwendung.

Einen eigenen Erfindungsgegenstand stellt die Verwendung eines erfindungsgemäßen proteolytischen Enzyms zur Aktivierung oder Deaktivierung von Inhaltsstoffen von Wasch- oder Reinigungsmitteln dar. Denn, wie bekannt ist, können Protein-Bestandteile von Wasch- oder Reinigungsmitteln durch das Einwirken einer Protease inaktiviert werden. Diesen ansonsten eher unerwünschten Effekt gezielt einzusetzen, ist ein Gegenstand der vorliegenden Erfindung. Ebenso ist es möglich, daß durch Proteolyse eine andere Komponente erst aktiviert wird, etwa, wenn sie ein Hybridprotein aus dem eigentlichen Enzym und dem dazu passenden Inhibitor darstellt, wie dies beispielsweise in der Anmeldung WO 00/01831 offenbart worden ist. Ein anderes Beispiel für eine solche Regulation ist die, bei der eine aktive Komponente zum Schutz oder zur Kontrolle seiner Aktivität in einem Material verkapselt vorliegt, das durch Proteolyse angegriffen wird. Erfindungsgemäße Proteine können somit zu Inaktivierungs-, Aktivierungs- oder Freisetzungsreaktionen verwendet werden.

Einen eigenen Erfindungsgegenstand stellt die Verwendung eines erfindungsgemäßen proteolytischen Enzyms zur biochemischen oder molekularbiologischen Analyse, insbesondere im Rahmen eines enzymatischen Analyseverfahrens dar. Erfindungsgemäß und nach Römpp, "Lexikon Chemie" (Version 2.0, Stuttgart/ New York: Georg Thieme Verlag, 1999) wird unter der enzymatischen Analyse jede biochemische Analytik verstanden, die sich spezifischer Enzyme oder Substrate bedient, um einerseits Substrate in ihrer Identität oder ihrer Konzentration oder andererseits Enzyme in Identität oder Aktivität zu bestimmen. Anwendungsgebiete sind alle mit der Biochemie verwandten Arbeitsgebiete. Eine bevorzugte Ausführungsform dieses Erfindungsgegenstands stellt die Verwendung zur Endgruppenbestimmung im Rahmen einer Sequenzanalyse dar.

Einen eigenen Erfindungsgegenstand stellt die Verwendung eines erfindungsgemäßen proteolytischen Enzyms zur Präparation, Reinigung oder Synthese von Naturstoffen oder biologischen Wertstoffen dar. So kann es im Verlauf der Aufreinigung von Naturstoffen oder biologischen Wertstoffen beispielsweise notwendig sein, diese von Proteinverunreinigungen zu befreien. Dabei kann es sich beispielweise um niedermolekulare Verbindungen, um alle Zellinhalts- oder Speicherstoffe oder um Proteine handeln. Dies ist sowohl im Labormaßstab, als auch in großtechnischer Dimension, beispielsweise nach der biotechnologischen Herstellung eines Wertstoffes durchführbar.

Die Verwendung eines erfindungsgemäßen proteolytischen Enzyms zur Synthese von Proteinen oder anderen niedermolekularen chemischen Verbindungen geschieht in Umkehrung der von ihnen natürlicherweise katalysierten Reaktion, beispielsweise dann, wenn Proteinfragmente miteinander verknüpft werden oder an eine nicht überwiegend aus Protein bestehende Verbindung Aminosäuren gebunden werden sollen. Derartige Verwendungsmöglichkeiten werden beispielsweise in der Anmeldung EP 380362 vorgestellt.

Einen eigenen Erfindungsgegenstand stellt die Verwendung eines erfindungsgemäßen proteolytischen Enzyms zur Behandlung von natürlichen Rohstoffen dar, wenn diese von Protein-Verunreinigungen befreit werden sollen. Hierunter sind in erster Linie nichtmikrobiologisch zu erhaltende Rohstoffe, etwa aus der Landwirtschaft zu verstehen.

Eine bevorzugte Ausführungsform stellt die Verwendung zur Oberflächenbehandlung, und ganz besonders in einem Verfahren zur Behandlung des wirtschaftlich bedeutenden Rohstoffs Leder dar. So werden im Verlauf des Gerbverfahrens, inbesondere im Schritt der Alkalischen Weiche (Römpp, "Lexikon Chemie", Version 2.0, Stuttgart/ New York: Georg Thieme Verlag, 1999) wasserlösliche Proteine mithilfe proteolytischer Enzyme aus dem Hautmaterial herausgelöst. Hierfür eignen sich, insbesondere wenn Alkalische Bedingungen herrschen und denaturierende Agentien anwesend sind, erfindungsgemäße Proteasen.

Einen eigenen Erfindungsgegenstand stellt die Verwendung eines erfindungsgemäßen proteolytischen Enzyms zur Gewinnung oder Behandlung von Rohmaterialien oder Zwischenprodukten in der Textilherstellung dar. Ein Beispiel dafür ist die Aufarbeitung von Baumwolle, die in einem als Schlichten bezeichneten Prozeß von den Kapselbestandteilen befreit werden muß; ein anderes die Behandlung von Wolle; ähnlich gestaltet sich auch die Verarbeitung von Rohseide. Enzymatische Verfahren sind vergleichbaren chemischen Verfahren besonders in ihrer Umweltverträglickeit überlegen.

In einer bevorzugten Ausführungsform werden erfindungsgemäße Proteine dazu verwendet, um Schutzschichten von Textilien, insbesondere Zwischenprodukten oder Werkstoffen zu entfernen oder ihre Oberfläche zu glätten, bevor sie in einem nachfolgenden Verarbeitungsschritt weiterbearbeitet werden.

In einem eigenen Erfindungsgegenstand werden erfindungsgemäße Proteine zur Behandlung von Textilrohstoffen oder zur Textilpflege, insbesondere zur Oberflächenbehandlung von Wolle oder Seide oder woll- oder seidenhaltigen Mischtextilien verwendet. Dies gilt sowohl für die Herstellung für derartige Textilien, als auch für die Pflege während des Gebrauchs, beispielsweise im Zusammenhang mit der Textilreinigung (siehe oben).

Einen eigenen Erfindungsgegenstand stellt die Verwendung eines erfindungsgemäßen proteolytischen Enzyms zur Behandlung von photographischen Filmen dar, insbesondere zur Entfernung von gelatinhaltigen oder ähnlichen Schutzschichten. Denn mit solchen Schutzschichten, insbesondere solchen aus Silbersalz-haltigen Gelatin-Emulsionen werden Filme, wie beispielsweise Röntgenfilme beschichtet, welche nach der Belichtung vom Trägermaterial abgelöst werden müssen. Hierfür können insbesondere unter Alkalischen oder leicht denaturierenden Reaktionsbedingungen erfindungsgemäße Proteasen verwendet werden.

Einen eigenen Erfindungsgegenstand stellt die Verwendung eines erfindungsgemäßen proteolytischen Enzyms zur Herstellung von Lebensmitteln oder von Futtermitteln dar. So werden Proteasen schon von alters her zur Lebensmittelherstellung verwendet. Ein Beispiel dafür ist die Verwendung von Lab für den Reifungsprozeß von Käse oder anderen Milchprodukten. Solche Prozesse können um ein erfindungsgemäßes Protein bereichert oder vollständig von ihm durchgeführt werden. Auch kohlenhydratreiche Lebensmittel oder Lebensmittelrohstoffe für Nicht-Emährungszwecke, wie beispielsweise Mehl oder Dextrin, können mit entsprechenden Proteasen behandelt werden, um sie von begleitenden Proteinen zu befreien. Auch für solche Anwendungen ist eine erfindungsgemäße Protease geeignet, insbesondere dann, wenn sie unter Alkalischen oder leicht denaturierenden Bedingungen durchgeführt werden sollen.

Entsprechendes gilt für die Futtermittelherstellung. Hier kann es neben einer vollständigen Befreiung von Proteinen auch von Interesse sein, die proteinhaltigen Ausgangsstoffe oder Stoffgemische mit Proteasen lediglich kurze Zeit zu behandeln, um sie für die Haustiere leichter verdaulich zu machen.

Einen eigenen Erfindungsgegenstand stellen kosmetische Mittel mit einem erfindungsgemäßen proteolytischen Enzym oder kosmetische Verfahren unter Einbeziehung eines erfindungsgemäßen proteolytischen Enzyms oder die Verwendung eines erfindungsgemäßen proteolytischen Enzyms zu kosmetischen Zwecken, insbesondere im Rahmen entsprechender Verfahren oder in entsprechenden Mitteln dar.

Denn Proteasen spielen auch im Zellerneuerungsprozeß der menschlichen Haut (Desquamation) eine entscheidende Rolle (T. Egelrud et al., Acta Derm. Venerol., Band 71 (1991), S. 471-474). Dementsprechend werden Proteasen auch als bioaktive Komponenten in Hautpflegemitteln verwendet, um den Abbau der in trockener Haut vermehrten Desmosomenstrukturen zu unterstützen, beispielsweise gemäß der Anmeldungen WO 95/07688 oder WO 99/18219. Der Einsatz von Subtilisin-Proteasen, insbesondere den bereits beschriebenen *B*. *lentus*-Alkalische Protease-Varianten für kosmetische Zwecke wird beispielsweise in WO 97/07770 beschrieben. Auch erfindungsgemäße Proteasen, insbesondere solche, die etwa nach Mutagenese oder durch Zugabe entsprechender, mit ihnen wechselwirkender Stoffe in ihrer Aktivität kontrolliert sind, eignen sich als aktive Komponenten in Haut- oder Haar-Reinigungs- oder Pflegemitteln. Besonders bevorzugt sind solche Präparationen dieser Enzyme, die wie oben beschrieben, beispielsweise durch Kopplung an makromolekulare Träger (vergleiche US 5230891) stabilisiert und/oder durch Punktmutationen an hochallergenen Positionen derivatisiert sind, so daß sie für den Menschen eine höhere Hautverträglichkeit aufweisen.

Dementsprechend wird auch die Verwendung derartiger proteolytischer Enzyme zu kosmetischen Zwecken in diesen Erfindungsgegenstand einbezogen, insbesondere in entsprechenden Mitteln, wie beispielsweise Shampoos, Seifen oder Waschlotionen, oder in Pfegemitteln, die beispielsweise in Form von Cremes angeboten werden. Auch die Verwendung in einem schälenden Arzneimittel ist in diesen Anspruch eingeschlossen.

### Beispiele

### Beispiel 1

### Erzeugung der erfindungsgemäßen Protease

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder in der internationalen Patentanmeldung WO 92/21760 angegeben sind.

### Konstruktion des Mutagenesevektors

Die Mutagenese wurde ausgehend von der Proteasevariante *B*. *lentus*-Alkalische Protease M131 durchgeführt. Diese Variante ist in WO 92/21760 beschrieben und der sie bildende Stamm gemäß dieser Anmeldung unter der Bezeichnung *Bacillus licheniformis* ATCC 68614 bei der American Type Culture Collection, Rockville, MD, USA, hinterlegt worden. In diesem Stamm ist auf dem in *Bacillus* replizierenden Plasmid pCB56M131 das Gen in einer Expressionskassette enthalten, bestehend aus dem Promotor, der Ribosomenbindungsstelle sowie dem ATG-Start-Codon und den 22 aminoterminalen Aminosäuren der alkalischen Protease aus *Bacillus licheniformis* ATCC 53926, fusioniert mit dem Prä-Pro-Protein und der mutierten Sequenz der *Bacillus lentus* DSM 5483-Alkalischen Protease. Die Variante *B*. *lentus*-Alkalische Protease M131 weist die folgenden Mutationen verglichen mit der nativen Sequenz auf: S3T, V41, A188P, V193M, V199I.

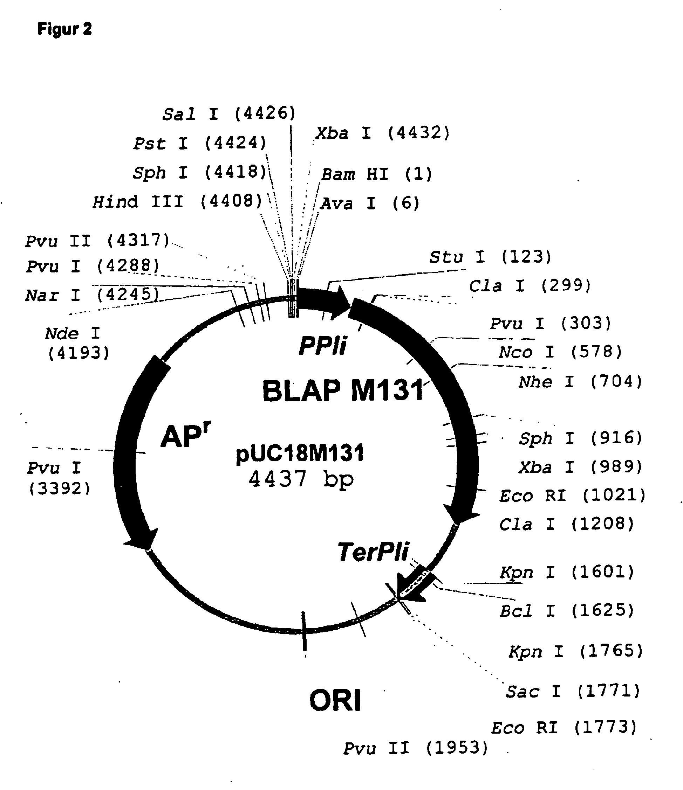

Zur Mutagenese wurde die gesamte Expressionskassette mittels der Restriktionsenzyme *Bam* HI und Sac I herausgeschnitten und in den ebenfalls mit *Bam* HI und Sac I geschnittenen Vektor pUC18 (Amersham Pharmacia Biotech, Freiburg) kloniert. Mit dem so erhaltenen Vektor pUC18M131 wurden dann die folgenden Mutageneseschritte durchgeführt. Der Vektor pUC18M131 ist in Figur 2 dargestellt. Das die Expressionskassette für *B. lentus*-Alkalische Protease M131 enthaltende DNA-Fragment ist in der SEQ ID NO. 1 dokumentiert; SEQ ID NO.2 zeigt die davon abgeleitete Aminosäuresequenz. Das in SEQ ID NO. 1 gezeigte *Bam* HI - *Sac* 1-Fragment erstreckt sich in dem in Figur 2 gezeigten Vektor pUC18M131 von den Positionen 1 bis 1771; die restlichen Vektorbereiche sind mit denen den Ausgangsplasmids pUC18 identisch.

### Mutagenese

Zunächst wurde die Originalsequenz der Alkalischen Protease von *Bacillus lentus* DSM 5483 in den Positionen 188 und 193 wiederhergestellt. Dazu wurde das QuikChange^{®}-Verfahren der Fa. Stratagene (La Jolla, CA, USA) nach den Angaben des Herstellers verwendet. Nach diesem System wurde unter Verwendung von jeweils zwei komplementären, die Mutation enthaltenden Primern ein mutiertes Plasmid in einer Polymerasereaktion erzeugt. Nach Verdau des Ausgangsplasmids mittels *Dpn*l wurde der Reaktionsansatz in *E. coli* XL-1 blue transformiert. Die erhaltenen Klone können gegebenenfalls mittels einer durch die Mutation eingefügten Restriktionsschnittstelle leicht erkannt werden, in jedem Fall ist die Überprüfung durch DNA-Sequenzierung mithilfe eines herkömmlichen Kits nach der Kettenabbruchmethode möglich.

Für die Umwandlung des für die Aminosäure in Position 188 codierenden Tripletts CCA (Prolin) in GCC (Alanin) wurden die beiden Primer 5'-TCA CAG TAT GGC GCC GGG CTT GAC ATT-3' sowie 5-AAT GTC AAG CCC GGC GCC ATA CTG TGA-3' verwendet. Diese enthalten unmittelbar neben der Mutation eine die Aminosäuresequenz nicht verändernde *Nar* I-Restriktionsschnittstelle.

Für die Umwandlung des für die Aminosäure in Position 193 codierenden Tripletts ATG (Methionin) in ATT (Isoleucin) wurden die beiden Primer 5'-GGG CTT GAC ATT GTG GCA CCC GGG GTA AAC-3' sowie 5'-GTT TAC CCC GGG TGC CAC AAT GTC AAG CCC-3' verwendet. Diese enthalten unmittelbar neben der Mutation eine die Aminosäuresequenz nicht verändernde *Xma* CI-Restriktionssite.

Ein das doppelt mutierte Plasmid enthaltender Klon lieferte dann die Matrize für die folgende Mutation des Tripletts in Position 211 TTA (Leucin) in GGA (Glycin). Dazu wurden die beiden komplementären Primer mit den Sequenzen 5'- ACG TAT GCT AGC GGA AAC GGT ACA TCG - 3' sowie 5'- CGA TGT ACC GTT TCC GCT AGC ATA CGT - 3' verwendet. Diese enthalten unmittelbar neben der Mutationstelle eine die Aminosäuresequenz nicht verändernde *Nhe* I-Restriktionssite. Die erhaltenen, mit *Nhe* I die erwarteten Fragmente ergebenden Klone wurden dann durch DNA-Sequenzierung überprüft.

Die DNA-Sequenz des für die gesamte Protease codierenden Gens der Mutante BLAP-S3T, V41, V199I, L211G ist im Sequenzprotokoll unter SEQ ID NO. 3 angegeben. Davon läßt sich die im Sequenzprotokoll unter SEQ ID NO. 4 angegebene Aminosäuresequenz ableiten. Diese *B. lentus*-Alkalische Protease-Variante wird wegen der vom Wildtyp-Enzym aus *B*. *lentus* DSM 5483 abweichenden Positionen als *B. lentus*-Alkalische Protease S3T/V4I/V199I/L211G bezeichnet.

### Expression der Mutante und Proteasepräparation

Die Expressionskassette mit der mutierten Sequenz wurde als *Bam* HI-*Sac* I-Fragment anstelle des in SEQ ID NO. 1 gezeigten Fragments in den Vektor pCB56M131 zurückkloniert und in *Bacillus subtilis* DB104 transformiert. Der Stamm *Bacillus subtilis* DB 104 weist den Genotyp *his, npr*R2, *npr*E18, *apr*A3 auf (Kawamura, F. und Doi, R. H. (1984), J. Bacteriol., Band 160, Seite 442-444). Die Transformation der DNA in Bacillus erfolgte nach der in WO91/02792 beschriebenen Variante der ursprünglich von Chang und Cohen entwickelten Protoplastenmethode (1979; Molec. Gen. Genet., Band 168, Seiten 111-115).

Dadurch erhaltene Protease-positive Klone wurden nach Überprüfung in 500 ml MLBSP-Medium (10 g/l Casiton; 20 g/l Trypton, 10 g/l Hefeextrakt, alle Fa. Becton Dickinson, Cockeysville; 5 g/l NaCl; 27 g/l Natrium-Succinat; 100 mg/l MgSO₄*7 H₂O; 75 mg/l CaCl₂*2 H₂O; 0,5 µM MnCl₂; 0,5 µM FeSO₄; 2 %(w/v) Glucose; 50 mM PIPES-Puffer (aus einer 1 M Stammlösung mit pH 7,2); 75 mM KPO₄(aus einer 1,5 M Stammlösung mit pH 7,0); pH = 7,0, eingestellt mit KOH - sowie 10 µg/ml Tetracyclin) in 2000 ml-Schüttelkolben für 72 h bei 37°C und 200 Umdrehungen pro Minute inkubiert. Der nach Abzentrifugieren der Zellen erhaltene Überstand wurde nach Bestimmung der Proteaseaktivität (nach der in Tenside, Band 7 (1970), S. 125-132 beschriebenen Methode) für die nachfolgenden Versuche verwendet.

### Beispiel 2

Für die folgenden beiden Beispiele wurden standardisiert verschmutze Textilien eingesetzt, die von der Eidgenössischen Material-Prüfungs- und -Versuchsanstalt, St. Gallen, Schweiz (EMPA), oder der Wäschereiforschungsanstalt, Krefeld, bezogen worden waren. In Beipiel 2 wurden folgende Anschmutzungen/Textilien verwendet: A (Blut/Milch/Ruß auf Baumwolle), B (Blut/Milch/Tusche auf Baumwolle), C (Blut/Milch/Tusche auf einem Polyester-Baumwolle-Mischgewebe) und D (Ei/Ruß auf Baumwolle).

Mit diesem Testmaterial wurden verschiedene Waschmittelrezepturen launderometrisch auf ihre Waschleistung hin untersucht. Dafür wurde jeweils ein Flottenverhältnis von 1:12 eingestellt und für 30 min bei einer Temperatur von 40°C gewaschen. Die Dosierung lag bei 5,88 g des jeweiligen Mittels pro I Waschflotte. Die Wasserhärte betrug 16° deutscher Härte.

Als Kontroll-Waschmittel diente eine Waschmittel-Basis-Rezeptur folgender Zusammensetzung (alle Angaben in Gewichts-Prozent): 4 % lineares Alkylbenzolsulfonat (Natrium-Salz), 4 % C₁₂-C₁₈-Fettalkoholsulfat (Natrium-Salz), 5,5 % C₁₂-C₁₈-Fettalkohol mit 7 EO, 1 % Natrium-Seife, 11 % Natriumcarbonat, 2,5 % amorphes Natriumdisilikat, 20 % Natriumperborat-Tetrahydrat, 5,5 % TAED, 25 % Zeolith A, 4,5 % Polycarboxylat, 0,5 % Phosphonat, 2,5 % Schauminhibitorgranulat, 5 % Natriumsulfat, Rest: Wasser, optischer Aufheller, Salze. Sie wurde für die verschiedenen Versuchsreihen so mit folgenden Proteasen versetzt, daß sich jeweils eine Endkonzentration von 2.250 PE an proteolytischer Aktivität pro I Waschflotte ergab: *B. lentus*-Alkalische Protease F49 (WO 95/23221; Hersteller: Fa. Biozym, Kundl, Österreich), Savinase^{®} (Fa. Novozymes A/S, Bagsværd, Dänemark), beziehungsweise die erfindungsgemäße Protease *B. lentus*-Alkalische Protease S3T/V4I/V199I/L211G.

Nach dem Waschen wurde der Weißheitsgrad der gewaschenen Textilien im Vergleich zu dem von Bariumsulfat gemessen, der auf 100 % normiert worden war. Die Messung erfolgte an einem Spektrometer Datacolor SF500-2 bei 460 nm (UV-Sperrfilter 3), 30 mm Blende, ohne Glanz, Lichtart D65, 10°, d/8°. Die erhaltenen Ergebnisse werden als Prozent Remission, das heißt als Prozentangaben im Vergleich zu Bariumsulfat zusammen mit den jeweiligen Anfangswerten in folgender Tabelle 3 zusammengestellt. Angegeben sind die Mittelwerte aus jeweils 4 Messungen. Sie erlauben einen unmittelbaren Rückschluß auf den Beitrag des enthaltenen Enzyms auf die Waschleistung des verwendeten Mittels.

**Tabelle 3:**

| **Basiswaschmittel mit** | **A** | **B** | **C** | **D** |
|---|---|---|---|---|
| Anfangswert | 22,9 | 13,0 | 11,3 | 26,4 |
| **Kontrolle ohne Protease** | 34,1 | 18,5 | 15,1 | 42,4 |
| ***B. lentus-*Alkalische Protease S3T/V4I/V199I/L211G** | 47,5 | 37,4 | 49,5 | 72,8 |
| ***B. lentus*-Alkalische Protease F49** | 40,1 | 28,6 | 26,8 | 71,3 |
| **Savinase^{®}** | 43,0 | 30,5 | 29,5 | 48,6 |
| Standardabw. | 0,7 | 0,7 | 1,2 | 0,9 |

Man erkennt, daß die erfindungsgemäße Protease an allen Anschmutzungen deutlich höhere Beiträge zu den Waschleistungen der jeweiligen Mittel zeigt als die herkömmlichen Proteasen *B. lentus-*Alkalische Protease F49 und Savinase^{®}.

### Beispiel 3

Zusätzlich zu den in Beipiel 2 angegebenen Anschmutzungen/Textilien wurde hier die Probe E (Blut auf Baumwolle) verwendet. Die Test-Textilien wurden auf dieselbe Weise wie in Beispiel 2 und mit entsprechenden Waschlösungen im Launderometer untersucht. Der einzige Unterschied gegenüber Beispiel 2 bestand darin, daß nun bei einer Temperatur von 60°C gewaschen wurde. Die Auswertung der Versuchsreihen erfolgte ebenfalls wie im vorangegangenen Beispiel beschrieben; folgende Tabelle 4 zeigt die Ergebnisse.

**Tabelle 4:**

| **Basiswaschmittel mit** | **A** | **B** | **C** | **D** | **E** |
|---|---|---|---|---|---|
| Anfangswert | 23,1 | 13,0 | 11,0 | 26,6 | 14,9 |
| **Kontrolle ohne Protease** | 34,2 | 18,6 | 15,3 | 44,3 | 52,8 |
| ***B. lentus*-Alkalische Protease S3T/V4I/V199I/L211G** | 46,2 | 46,2 | 60,3 | 72,5 | 64,0 |
| ***B. lentus*-Alkalische Protease F49** | 30,7 | 30,7 | 32,2 | 72,4 | 55,3 |
| **Savinase^{®}** | 35,9 | 35,9 | 36,4 | 56,5 | 54,0 |
| Standardabw. | 0,9 | 0,9 | 1,8 | 0,8 | 1,3 |

Wie dieses Ergebnis zeigt, ist die erfindungsgemäße Alkalische Protease S3T/V4I/V199I/L211G den anderen, für Waschmitteln etablierten Proteasen *B. lentus*-Alkalische Protease F49 und Savinase^{®} auch bei der Waschtemperatur von 60°C in ihrem Beitrag zur Waschleistung des jeweiligen Mittels überlegen oder innerhalb der Fehlergrenze zumindest ebenbürtig.

### Beispiel 4

Gefäße mit harten, glatten Oberflächen wurden standardisiert mit (A) Weichei, (B) Ei/ Milch, (C) Mix-Stärke und (D) Hackfleisch versehen und bei 45°C mit dem Normalprogramm einer Haushaltsgeschirrspülmaschine Typ Miele^{®} G 676 gespült. Pro Spülgang wurden 20 g Spülmittel verwendet; die Wasserhärte betrug 16° deutscher Härte.

Als Spülmittel diente folgende Basis-Rezeptur (alle Angaben jeweils in Gewichts-Prozent): 55 % Natriumtripolyphosphat (berechnet als wasserfrei), 4 % amorphes Natriumdisilikat (berechnet als wasserfrei), 22 % Natriumcarbonat, 9 % Natriumperborat, 2 % TAED, 2 % nichtionisches Tensid, Rest: Wasser, Farbstoffe, Parfüm. Diese Basis-Rezeptur wurde für die verschiedenen Versuche aktivitätsgleich so mit den verschiedenen Proteasen *B*. *lentus*-Alkalische Protease F49, Savinase^{®}, beziehungsweise der erfindungsgemäßen Protease-Variante *B.lentus*-Alkalische Protease S3T/V4I/V199I/L211G versetzt, daß sich jeweils eine Aktivität von 10.000 PE pro Spülgang ergab. Dies entsprach jeweils ca. 0,1 mg Protease-Protein pro g des Reinigungsmittelkonzentrats.

Nach dem Spülen wurde der Abtrag der Anschmutzungen A bis C gravimetrisch in Prozent ermittelt. Dafür wurde die Differenz aus dem Gewicht des verschmutzten und anschließend gespülten Gefäßes und dem Anfangsgewicht des Gefäßes in Relation zu der Gewichtsdifferenz des nicht gespülten Gefäßes zum Anfangsgewicht gesetzt. Diese Relation kann als Prozent Abtrag angesehen werden. Für die Anschmutzung D wurde nach dem Spülen eine visuelle Brwertung nach einer Skala von 0 (= unverändert, das heißt sehr starke Anschmutzung) bis 10 (= keinerlei Anschmutzung erkennbar) vorgenommen. Die erhaltenen Ergebnisse werden in folgender Tabelle 5 zusammengestellt. Angegeben sind dort die Mittelwerte aus jeweils 9 Messungen. Sie erlauben einen unmittelbaren Rückschluß auf den Beitrag des enthaltenen Enzyms auf die Reinigungsleistung des verwendeten Mittels.

**Tabelle 5:**

| **Basisspülmittel mit** | **A** | **B** | **C** | **D** |
|---|---|---|---|---|
| | % Abtrag | % Abtrag | % Abtrag | Note |
| ***B*. *lentus*-Alkalische Protease S3T/V4I/V199I/L211G** | 25,2 | 27,3 | 69,3 | 9,8 |
| ***B. lentus*-Alkalische Protease F49** | 26,2 | 22,4 | 65,2 | 7,6 |
| **Savinase^{®}** | 12,5 | 12,0 | 63,3 | 8,4 |

Diese Ergebnisse zeigen, daß die erfindungsgemäße *B*. *lentus*-Alkalische Protease S3T/V4I/N199I/L211G hinsichtlich ihres Beitrags zur Reinigungsleistung in maschinellen Geschirrspülmitteln den anderen getesteten Proteasen überlegen, zumindest aber ebenbürtig ist; und dies bereits bei einer vergleichsweise niedrigen eingesetzten Aktivität.

### Beispiel 5

Wie im vorangegangenen Beispiel wurden Gefäße standardmäßig mit denselben Anschmutzungen versehen und auf die gleiche Weise mit den jeweils gleichen Reinigungsmittelrezepturen gespült. Der einzige Unterschied bestand darin, daß jeweils 20.000 PE an den jeweiligen Proteasen eingesetzt wurden. Dies entsprach jeweils ca. 0,2 mg Protease im Reinigungsmittelkonzentrat. Die auf die gleiche Weise wie in Beispiel 4 erhaltenen Meßergebnisse sind in folgender Tabelle 6 zusammengestellt.

**Tabelle 6:**

| **Basisspülmittel mit** | **A** | **B** | **D** |
|---|---|---|---|
| | % Abtrag | % Abtrag | Note |
| ***B. lentus*-Alkalische Protease S3T/V4I/V199I/L211G** | 35,4 | 37,6 | 9,4 |
| ***B. lentus*-Alkalische Protease F49** | 33,2 | 32,7 | 9,1 |
| **Savinase^{®}** | 12,4 | 14,0 | 8,7 |

Auch bei höheren eingesetzten Protease-Aktivitäten zeigt sich der höhere Beitrag der erfindungsgemäßen Protease zur Gesamtreinigungsleistung des betreffenden Mittels gegenüber den für maschinelle Geschirrspülmittel etablierten Proteasen *B. lentus-*Alkalische Protease F49 und Savinase^{®}.

### Beschreibung der Figuren

- **Figur 1**:: Alignment der Aminosäuresequenzen der erfindungsgemäßen *B. lentus*-Alkalische Protease-Variante mit den wichtigsten bekannten Subtilisinen, jeweils in der maturen, das heißt prozessierten Form.
- Darin bedeuten: erf.-gem. Variante: Erfindungsgemäße *B. lentus*-Alkalische Protease-Variante S3T/V4I/V199I/L211G;
- Subtilisin 309: Subtilisin aus *Bacillus lentus* gemäß WO 89/06279;
- Subtilisin PB92: Subtilisin aus *Bacillus nov.* spec. 92 gemäß EP 283075;
- Subtilisin Carlsberg (1968),: Subtilisin aus *Bacillus licheniformis* gemäß E. L. Smith et al. J. Biol. Chem., Volume 243, S. 2184-2191;
- Subtilisin BPN': Subtilisin aus *Bacillus amyloliquefaciens* gemäß J. A. Wells et al. (1983), Nucleic Acids Research, Volume 11, S. 7911-7925;
- Consensus: In der Mehrzahl der angegebenen Sequenzen übereinstimmende Positionen.
- **Figur 2:**: Mutagenesevektor pUC18M131. Das in SEQ ID NO. 1 gezeigte *Bam* HI - *Sac* I-Fragment erstreckt sich darin von den Positionen 1 bis 1771; die restlichen Vektorbereiche sind mit denen den Ausgangsplasmids pUC18 (Amersham Pharmacia Biotech, Freiburg) identisch.

## Patentansprüche

1. Alkalische Protease vom Subtilisin-Typ, **dadurch gekennzeichnet, daß** sie gemäß der Zählung des Subtilisins aus *Bacillus lentus* DSM 5483 in Position 199 Isoleucin und in Position 211 Glycin aufweist und zusätzlich eine der Aminosäuren Threonin in Position 3 oder Isoleucin in Position 4.

2. Alkalische Protease vom Subtilisin-Typ gemäß Anspruch 1, **dadurch gekennzeichnet, daß** sie gemäß der Zählung des Subtilisins aus *Bacillus lentus* DSM 5483 in Position 3 Threonin, in Position 4 Isoleucin, in Position 199 Isoleucin und in Position 211 Glycin aufweist.

3. Alkalische Protease gemäß einem der Ansprüche 1 bis 2, **dadurch gekennzeichnet, daß** sie ein von einem Bacillus, insbesondere von *Bacillus lentus,* natürlicherweise gebildetes oder ein von solch einem Bacillus abgeleitetes Subtilisin ist.

4. Alkalische Protease gemäß Anspruch 3, **dadurch gekennzeichnet, daß** sie ein von *Bacillus lentus* DSM 5483 natürlicherweise gebildetes oder ein von solch einem abgeleitetes Subtilisin ist.

5. Alkalische Protease gemäß Anspruch 4, wobei es sich um die *B. lentus-*Alkalische Protease S3T/V4I/V199I/L211G gemäß der in Figur 1 (erf.-gem. Variante) angegebenen Aminosäuresequenz handelt.

6. Alkalische Protease gemäß einem der Ansprüche 1 bis 5, abgeleitet von einer Protease gemäß einem der Ansprüche 1 bis 5, insbesondere durch Fragmentierung oder Deletionsmutagenese, durch Insertionsmutagenese, durch Substitutionsmutagenese oder durch Fusion mindestens eines Teils mit mindestens einem anderen Protein, wobei die darin genannten Punktmutationen beibehalten werden.

7. Alkalische Protease gemäß einem der Ansprüche 1 bis 6, **dadurch gekennzeichnet, daß** sie zusätzlich derivatisiert ist, insbesondere über chemische Modifikation, durch Kopplung nieder- oder höhermolekularer chemischer Verbindungen und/oder durch Vergesellschaftung mit anderen Stoffen.

8. Alkalische Protease gemäß Anspruch 6 oder 7, **dadurch gekennzeichnet, daß** sie gegenüber dem Ausgangsmolekül beziehungsweise nicht-derivatisierten Molekül erhöhte proteolytische Aktivität, und ganz besonders eine verbesserte Leistung aufweist.

9. Alkalische Protease gemäß einem der Ansprüche 6 bis 8, **dadurch gekennzeichnet, daß** sie zusätzlich stabilisiert ist, insbesondere durch gemeinsame Präparation mit anderen Stoffen, insbesondere mit Protease-Inhibitoren, durch Bildung eines Fusionsproteins mit einem Protease-Inhibitor, durch stabilisierende Mutationen und/oder durch Kopplung an ein Polymer.

10. Nukleinsäure, die für eine der in den Ansprüchen 1 bis 9 bezeichneten Proteasen codiert.

11. Für eine Subtilisin-Protease codierende Nukleinsäure, deren Nukleotidsequenz mit der in SEQ ID NO. 3 angegebenen Nukleotidsequenz übereinstimmt, besonders in den Bereichen, die für 199 Isoleucin und 211 Glycin und ganz besonders in den Bereichen, die für 3 Threonin, 4 Isoleucin, 199 Isoleucin und 211 Glycin codieren.

12. Vektor, der einen in den Ansprüchen 10 oder 11 bezeichneten Nukleinsäurebereich enthält.

13. Klonierungsvektor gemäß Anspruch 12.

14. Expressionsvektor gemäß Anspruch 12, der insbesondere die Biosynthese einer der in den Ansprüchen 1 bis 9 bezeichneten Proteasen ermöglicht.

15. Zelle, die einen Vektor nach einem der Ansprüche 12 bis 14 enthält.

16. Wirtszelle, die eine der in Ansprüchen 1 bis 9 bezeichneten Proteasen exprimiert oder zu deren Expression angeregt werden kann, insbesondere unter Einsatz eines Expressionsvektors gemäß Anspruch 14.

17. Wirtszelle gemäß Anspruch 16, **dadurch gekennzeichnet, daß** sie ein Bakterium ist, insbesondere eines, das das gebildete Protein ins umgebende Medium sekretiert.

18. Wirtszelle gemäß Anspruch 17, **dadurch gekennzeichnet, daß** sie ein Bakterium der Gattung Bacillus, insbesondere der Species *Bacillus lentus, Bacillus licheniformis, Bacillus amyloliquefaciens, Bacillus subtilis* oder *Bacillus alcalophilus* ist.

19. Wirtszelle gemäß Anspruch 15 oder 16, **dadurch gekennzeichnet, daß** sie eine eukaryontische Zelle ist, insbesondere eine, die das gebildete Protein posttranslational modifiziert.

20. Verfahren zur Herstellung eines proteolytischen Enzyms nach einem der Ansprüche 1 bis 9 unter Verwendung einer Wirtszelle gemäß einem der Ansprüche 15 bis 19 und/oder unter Verwendung eines Vektors gemäß einem der Ansprüche 12 bis 14 und/oder unter Verwendung einer Nukleinsäure gemäß einem der Ansprüche 10 oder 11.

21. Mittel, **dadurch gekennzeichnet, daß** es ein proteolytisches Enzym nach einem der Ansprüche 1 bis 9 enthält, insbesondere Wasch- oder Reinigungsmittel, ganz besonders in einer Menge von 2 µg bis 20 mg pro g des Mittels.

22. Mittel nach Anspruch 21, **dadurch gekennzeichnet, daß** es zusätzlich weitere Enzyme, insbesondere andere Proteasen, Amylasen, Cellulasen, Hemicellulasen und/oder Lipasen enthält.

23. Mittel zur Behandlung von Textilrohstoffen oder zur Textilpflege, **dadurch gekennzeichnet, daß** es allein oder neben anderen aktiven Inhaltsstoffen ein proteolytisches Enzym nach einem der Ansprüche 1 bis 9 enthält, insbesondere für Fasern oder Textilien mit natürlichen Bestandteilen und ganz besonders für solche mit Wolle oder Seide.

24. Verfahren zur maschinellen Reinigung von Textilien oder von harten Oberflächen, **dadurch gekennzeichnet, daß** in wenigstens einem der Verfahrensschritte ein proteolytisches Enzym nach einem der Ansprüche 1 bis 9 aktiv wird, vorzugsweise in einer Menge von 40 µg bis 4g, besonders bevorzugt von 400 µg bis 400 mg pro Anwendung.

25. Verfahren zur Behandlung von Textilrohstoffen oder zur Textilpflege, **dadurch gekennzeichnet, daß** in wenigstens einem der Verfahrensschritte ein proteolytisches Enzym nach einem der Ansprüche 1 bis 9 aktiv wird, insbesondere für Textilrohstoffe oder Textilien mit natürlichen Bestandteilen, insbesondere für solche mit Wolle oder Seide.

26. Verwendung eines proteolytischen Enzyms nach einem der Ansprüche 1 bis 9 zur Reinigung von Textilien oder von harten Oberflächen, vorzugsweise in einer Menge von 40 µg bis 4 g, besonders bevorzugt von 400 µg bis 400 mg pro Anwendung.

27. Verwendung eines proteolytischen Enzyms nach einem der Ansprüche 1 bis 9 zur Aktivierung oder Deaktivierung von Inhaltsstoffen von Wasch- oder Reinigungsmitteln.

28. Verwendung eines proteolytischen Enzyms nach einem der Ansprüche 1 bis 9 zur biochemischen Analyse oder zur Synthese von niedermolekularen Verbindungen oder von Proteinen.

29. Verwendung eines proteolytischen Enzyms nach einem der Ansprüche 1 bis 9 zur Präparation, Reinigung oder Synthese von Naturstoffen oder biologischen Wertstoffen.

30. Verwendung eines proteolytischen Enzyms nach einem der Ansprüche 1 bis 9 zur Behandlung von natürlichen Rohstoffen, insbesondere zur Oberflächenbehandlung, ganz besonders in einem Verfahren zur Behandlung von Leder.

31. Verwendung eines proteolytischen Enzyms nach einem der Ansprüche 1 bis 9 zur Gewinnung oder Behandlung von Rohmaterialien oder Zwischenprodukten in der Textilherstellung, insbesondere zum Entfernen von Schutzschichten auf Geweben.

32. Verwendung eines proteolytischen Enzyms nach einem der Ansprüche 1 bis 9 zur Behandlung von Textilrohstoffen oder zur Textilpflege, insbesondere zur Behandlung von Wolle oder Seide oder woll- oder seidenhaltigen Mischtextilien.

33. Verwendung eines proteolytischen Enzyms nach einem der Ansprüche 1 bis 9 zur Behandlung von photographischen Filmen, insbesondere zur Entfernung von gelatinhaltigen oder ähnlichen Schutzschichten.

34. Verwendung eines proteolytischen Enzyms nach einem der Ansprüche 1 bis 9 zur Herstellung von Lebensmitteln oder von Futtermitteln.

35. Kosmetika mit einem proteolytischen Enzym nach einem der Ansprüche 1 bis 9 oder kosmetische Verfahren unter Einbeziehung eines proteolytischen Enzyms nach einem der Ansprüche 1 bis 9 oder die Verwendung eines proteolytischen Enzyms nach einem der Ansprüche 1 bis 9 zu kosmetischen Zwecken, insbesondere im Rahmen entsprechender Verfahren oder in entsprechenden Mitteln.

## Claims

1. Alkaline protease of the subtilisin type, **characterized in that**, according to the numbering of *Bacillus lentus* DSM 5483 subtilisin, it has isoleucine at position 199 and glycine at position 211 and additionally either of the amino acids threonine at position 3 or isoleucine at position 4.

2. Alkaline protease of the subtilisin type according to Claim 1, **characterized in that**, according to the numbering of *Bacillus lentus* DSM 5483 subtilisin, it has threonine at position 3, isoleucine at position 4, isoleucine at position 199 and glycine at position 211.

3. Alkaline protease according to either of Claims 1 and 2, **characterized in that** it is a subtilisin naturally produced by a Bacillus, in particular *Bacillus lentus,* or derived from such a Bacillus.

4. Alkaline protease according to Claim 3, **characterized in that** it is a subtilisin naturally produced by or derived from *Bacillus lentus* DSM 5483.

5. Alkaline protease according to Claim 4, which is *B. lentus* alkaline protease S3T/V4I/V199I/L211G according to the amino acid sequence indicated in Figure 1 (inventive variant).

6. Alkaline protease according to any of Claims 1 to 5, derived from a protease according to any of Claims 1 to 5, in particular by fragmentation or deletion mutagenesis, by insertion mutagenesis, by substitution mutagenesis or by fusion of at least one part to at least one other protein wherein the point mutations specified therein are retained.

7. Alkaline protease according to any of Claims 1 to 6, **characterized in that** it is additionally derivatized, in particular by way of chemical modification, by coupling of low molecular-weight or high molecular-weight chemical compounds and/or by association with other substances.

8. Alkaline protease according to Claim 6 or 7, **characterized in that** it has increased proteolytic activity, and very particularly an enhanced performance, compared to the starting molecule and the non-derivatized molecule.

9. Alkaline protease according to any of Claims 6 to 8, **characterized in that** it is additionally stabilized, in particular by being prepared together with other substances, in particular with protease inhibitors, by forming a fusion protein with a protease inhibitor, by stabilizing mutations and/or by coupling to a polymer.

10. Nucleic acid coding for any of the proteases as defined in Claims 1 to 9.

11. Nucleic acid coding for a subtilisin protease, whose nucleotide sequence corresponds to the nucleotide sequence indicated in SEQ ID NO.3, in particular in the regions coding for 199 isoleucine and 211 glycine and very particularly in the regions coding for 3 threonine, 4 isoleucine, 199 isoleucine and 211 glycine.

12. Vector comprising a nucleic acid region as defined in Claims 10 or 11.

13. Cloning vector according to Claim 12.

14. Expression vector according to Claim 12, which in particular makes possible the biosynthesis of any of the proteases defined in Claims 1 to 9.

15. Cell comprising a vector according to any of Claims 12 to 14.

16. Host cell which expresses or can be induced to express any of the proteases as defined in Claims 1 to 9, in particular by using an expression vector according to Claim 14.

17. Host cell according to Claim 16, **characterized in that** it is a bacterium, in particular one which secretes the protein produced in the surrounding medium.

18. Host cell according to Claim 17, **characterized in that** it is a bacterium of the genus Bacillus, in particular of the species *Bacillus lentus, Bacillus licheniformis, Bacillus amyloliquefaciens, Bacillus subtilis* or *Bacillus alcalophilus.*

19. Host cell according to Claim 15 or 16, **characterized in that** it is an eukaryotic cell, in particular one which modifies posttranslationally the protein produced.

20. Method for preparing a proteolytic enzyme according to any of Claims 1 to 9, using a host cell according to any of Claims 15 to 19 and/or using a vector according to any of Claims 12 to 14 and/or using a nucleic acid according to either of Claims 10 and 11.

21. Agent, **characterized in that** it contains a proteolytic enzyme according to any of Claims 1 to 9, in particular a detergent or cleaning agent, very particularly in an amount of from 2 µg to 20 mg per g of said agent.

22. Agent according to Claim 21, **characterized in that** it additionally contains further enzymes, in particular other proteases, amylases, cellulases, hemicellulases and/or lipases.

23. Agent for the treatment of textile raw materials or for textile care, **characterized in that** it contains, solely or in addition to other active ingredients, a proteolytic enzyme according to any of Claims 1 to 9, in particular for fibres or textiles containing natural components and very particularly for those containing wool or silk.

24. Method for machine-cleaning textiles or hard surfaces, **characterized in that** in at least one of the method steps a proteolytic enzyme according to any of Claims 1 to 9 becomes active, preferably in an amount of from 40 µg to 4 g, particularly preferably from 400 µg to 400 mg, per application.

25. Method for the treatment of textile raw materials or for textile care, **characterized in that** in at least one of the method steps a proteolytic enzyme according to any of Claims 1 to 9 becomes active, in particular for textile raw materials or textiles containing natural components, in particular for those containing wool or silk.

26. Use of a proteolytic enzyme according to any of Claims 1 to 9 for cleaning textiles or hard surfaces, preferably in an amount of from 40 µg to 4 g, particularly preferably from 400 µg to 400 mg, per application.

27. Use of a proteolytic enzyme according to any of Claims 1 to 9 for activating or deactivating ingredients of detergents or cleaning agents.

28. Use of a proteolytic enzyme according to any of Claims 1 to 9 for biochemically analyzing or for synthesizing low molecular weight compounds or proteins.

29. Use of a proteolytic enzyme according to any of Claims 1 to 9 for preparing, purifying or synthesizing natural substances or biological valuable substances.

30. Use of a proteolytic enzyme according to any of Claims 1 to 9 for the treatment of natural raw materials, in particular for the treatment of surfaces, very particularly in a method for the treatment of leather.

31. Use of a proteolytic enzyme according to any of Claims 1 to 9 for the obtainment or treatment of raw materials or intermediates in the manufacture of textiles, in particular for removing protective layers on fabrics.

32. Use of a proteolytic enzyme according to any of Claims 1 to 9 for the treatment of textile raw materials or for textile care, in particular for the treatment of wool or silk or of wool- or silk-containing mixed textiles.

33. Use of a proteolytic enzyme according to any of Claims 1 to 9 for the treatment of photographic films, in particular for removing gelatin-containing or similar protective layers.

34. Use of a proteolytic enzyme according to any of Claims 1 to 9 for preparing food or animal feed.

35. Cosmetic containing a proteolytic enzyme according to any of Claims 1 to 9 or a cosmetic method including a proteolytic enzyme according to any of Claims 1 to 9 or the use of a proteolytic enzyme according to any of Claims 1 to 9 for cosmetic purposes, in particular within the framework of corresponding methods or in corresponding agents.

## Revendications

1. Protéase alcaline du type subtilisine, **caractérisée en ce qu'**elle présente, conformément au comptage de la subtilisine de *Bacillus lentus* DSM 5483, de l'isoleucine en position 199 et de la glycine en position 211 et en outre un des acides aminés thréonine en position 3 ou isoleucine en position 4.

2. Protéase alcaline du type subtilisine selon la revendication 1, **caractérisée en ce qu'**elle présente, conformément au comptage de la subtilisine de *Bacillus lentus* DSM 5483, en position 3 de la thréonine, en position 4 de l'isoleucine, en position 199 de l'isoleucine et en position 211 de la glycine.

3. Protéase alcaline selon l'une quelconque des revendications 1 à 2, **caractérisée en ce qu'**elle représente une subtilisine d'un Bacillus, en particulier de *Bacillus lentus* formée naturellement ou une subtilisine qui dérive d'un tel Bacillus.

4. Protéase alcaline selon la revendication 3, **caractérisée en ce qu'**elle représente une subtilisine de *Bacillus lentus* DSM 5483 formée naturellement ou une subtilisine qui en dérive.

5. Protéase alcaline selon la revendication 4, **caractérisée en ce qu'**il s'agit de la protéase alcaline de *B*. *lentus* S3T/V4I/V199I/L211G conformément à la séquence d'acides aminés indiquée en figure 1 (le cas échéant, conformément à une variante).

6. Protéase alcaline selon l'une quelconque des revendications 1 à 5, qui dérive d'une protéase selon l'une quelconque des revendications 1 à 5, en particulier par fragmentation ou mutagenèse de délétion, par mutagenèse d'insertion, par mutagenèse de substitution ou par fusion d'au moins une partie avec au moins une autre protéine, les mutations ponctuelles qui y sont mentionnées étant conservées.

7. Protéase alcaline selon l'une quelconque des revendications 1 à 6, **caractérisée en ce qu'**elle est en outre dérivée, en particulier via une modification chimique, par couplage de composés chimiques à bas poids moléculaire ou à poids moléculaire élevé et/ou par association avec d'autres substances.

8. Protéase alcaline selon la revendication 6 ou 7, **caractérisée en ce qu'**elle présente, par rapport à la molécule de départ, respectivement par rapport à une molécule non dérivée, une plus grande activité protéolytique, et de manière tout à fait particulière, un meilleur rendement.

9. Protéase alcaline selon l'une quelconque des revendications 6 à 8, **caractérisée en ce qu'**elle est en outre stabilisée, en particulier via une préparation commune avec d'autres substances, en particulier avec des inhibiteurs de protéases, par formation d'une protéine de fusion avec un inhibiteur de protéases, via des mutations stabilisantes et/ou par couplage à un polymère.

10. Acide nucléique qui code pour une des protéases indiquées aux revendications 1 à 9.

11. Acide nucléique codant pour une subtilisine-protéase dont la séquence nucléotidique concorde avec la séquence nucléotidique indiquée dans SEQ ID NO: 3, en particulier dans les domaines qui codent l'isoleucine 199 et la glycine 211 et de manière tout à fait particulière dans les domaines qui codent la thréonine 3, l'isoleucine 4, l'isoleucine 199 et la glycine 211.

12. Vecteur qui contient un domaine d'acide nucléique indiqué dans la revendication 10 ou 11.

13. Vecteur de clonage selon la revendication 12.

14. Vecteur d'expression selon la revendication 12, qui rend en particulier possible la biosynthèse d'une des protéases indiquées aux revendications 1 à 9.

15. Cellule qui contient un vecteur selon l'une quelconque des revendications 12 à 14.

16. Cellule hôte qui exprime une des protéases indiquées aux revendications 1 à 9 ou qui peut être incitée à une telle expression, en particulier par la mise en oeuvre d'un vecteur d'expression selon la revendication 14.

17. Cellule hôte selon la revendication 16, **caractérisée en ce qu'**elle représente une bactérie, en particulier une bactérie qui sécrète la protéine obtenue dans le milieu environnant.

18. Cellule hôte selon la revendication 17, **caractérisée en ce qu'**elle représente une bactérie du genre Bacillus, en particulier de l'espèce *Bacillus lentus, Bacillus licheniformis, Bacillus amyloliquefaciens, Bacillus subtilis* ou *Bacillus alcalophilus.*

19. Cellule hôte selon la revendication 15 ou 16, **caractérisée en ce qu'**elle représente une cellule eucaryote, en particulier une cellule eucaryote qui soumet la protéine obtenue à une modification posttraductionnelle.

20. Procédé pour la préparation d'une enzyme protéolytique selon l'une quelconque des revendications 1 à 9 en utilisant une cellule hôte selon l'une quelconque des revendications 15 à 19 et/ou en utilisant un vecteur selon l'une quelconque des revendications 12 à 14 et/ou en utilisant un acide nucléique selon l'une quelconque des revendications 10 ou 11.

21. Agent, **caractérisé en ce qu'**il contient une enzyme protéolytique selon l'une quelconque des revendications 1 à 9, en particulier agent de lavage ou de nettoyage, de manière tout à fait particulière en une quantité de 2 µg à 20 mg par g de l'agent.

22. Agent selon la revendication 21, **caractérisé en ce qu'**il contient en outre d'autres enzymes, en particulier d'autres protéases, amylases, cellulases, hémicellulases et/ou lipases.

23. Agent pour le traitement de matières premières textiles ou pour l'entretien des textiles, **caractérisé en ce qu'**il contient à titre individuel ou à côté d'autres constituants actifs, une enzyme protéolytique selon l'une quelconque des revendications 1 à 9, en particulier pour des fibres ou des textiles comprenant des constituants naturels et, de manière tout à fait particulière, pour des fibres ou des textiles comprenant de la laine ou de la soie.

24. Procédé pour le nettoyage mécanique de textiles ou de surfaces dures, **caractérisé en ce que**, dans au moins une des étapes opératoires, une enzyme protéolytique selon l'une quelconque des revendications 1 à 9 devient active, de préférence en une quantité de 40 µg à 4 g, de manière particulièrement préférée de 400 µg à 400 mg par utilisation.

25. Procédé pour le traitement de matières premières textiles ou pour l'entretien des textiles, **caractérisé en ce que**, dans au moins une des étapes opératoires, une enzyme protéolytique selon l'une quelconque des revendications 1 à 9 devient active, en particulier pour des matières premières textiles ou des textiles comprenant des constituants naturels, en particulier pour des matières premières textiles ou des textiles comprenant de la laine ou de la soie.

26. Utilisation d'une enzyme protéolytique selon l'une quelconque des revendications 1 à 9, pour le nettoyage de textiles ou de surfaces dures, de préférence en une quantité de 40 µg à 4 g, de manière particulièrement préférée de 400 µg à 400 mg par utilisation.

27. Utilisation d'une enzyme protéolytique selon l'une quelconque des revendications 1 à 9, pour l'activation ou la désactivation de constituants d'agents de lavage ou de nettoyage.

28. Utilisation d'une enzyme protéolytique selon l'une quelconque des revendications 1 à 9, pour l'analyse biochimique ou pour la synthèse de composés à bas poids moléculaire ou de protéines.

29. Utilisation d'une enzyme protéolytique selon l'une quelconque des revendications 1 à 9, pour la préparation, la purification ou la synthèse de substances naturelles ou de substances biologiques de valeur.

30. Utilisation d'une enzyme protéolytique selon l'une quelconque des revendications 1 à 9, pour le traitement de matières premières naturelles, en particulier pour le traitement superficiel, de manière tout à fait particulière dans un procédé pour le traitement du cuir.

31. Utilisation d'une enzyme protéolytique selon l'une quelconque des revendications 1 à 9, pour l'obtention ou le traitement de matières premières ou de produits intermédiaires dans la confection des textiles, en particulier pour éliminer des couches de protection sur des tissus.

32. Utilisation d'une enzyme protéolytique selon l'une quelconque des revendications 1 à 9, pour le traitement de matières premières textiles ou pour l'entretien des textiles, en particulier pour le traitement de la laine ou de la soie ou encore de textiles mixtes contenant de la laine ou de la soie.

33. Utilisation d'une enzyme protéolytique selon l'une quelconque des revendications 1 à 9, pour le traitement de films photographiques, en particulier pour éliminer des couches de protection contenant de la gélatine ou des couches de protection analogues.

34. Utilisation d'une enzyme protéolytique selon l'une quelconque des revendications 1 à 9, pour la préparation de denrées alimentaires ou d'aliments pour animaux.

35. Produit cosmétique comprenant une enzyme protéolytique selon l'une quelconque des revendications 1 à 9 ou procédé cosmétique avec incorporation d'une enzyme protéolytique selon l'une quelconque des revendications 1 à 9, ou bien utilisation d'une enzyme protéolytique selon l'une quelconque des revendications 1 à 9 à des fins cosmétiques, en particulier dans le cadre de procédés correspondants ou dans des agents correspondants.
